# EUROPEAN PATENT APPLICATION

(11) **EP 1 484 324 A1**
(43) Date of publication of application: **08.12.2004**
(21) Application number: 03012865.6
(22) Date of filing: 06.06.2003
(51) Int. Cl.: C07D 251/18, A01N 43/68

(54) **Substituted 2,4-diamino-1,3,5-triazines, processes for their preparation and their use as herbicides and plant growth regulators**

(71) Applicant: Bayer CropScience GmbH, 65929 Frankfurt (DE)
(72) Inventor: Giencke, Wolfgang, Dr., 65719 Hofheim (DE); Dietrich, Hansjörg, Dr., 65719 Hofheim (DE); Bieringer, Hermann, Dr., 65817 Eppstein (DE); Hills, Martin, 65510 Idstein (DE); Feucht, Dieter, Dr., 65760 Eschborn (DE)

(57) **Abstract**

Compounds of the formula (I) or salts thereof, 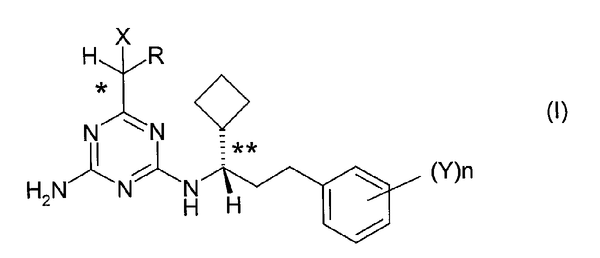 in which
the chiral carbon atom alpha to the amino group of the phenylalkylamino radical marked ** is in the S-configuration, as indicated in formula (I) whilst the chiral carbon atom to which the R and X radicals are bonded marked * can be in either the R or S configuration, and in which
- R: is (C₁-C₆)alkyl, (C₁-C₆)haloalkyl, [(C₁-C₆)alkoxy](C₁-C₆)alkyl, (C₂-C₆)alkenyl, (C₂-C₆)haloalkenyl, (C₁-C₆)alkoxy, (C₁-C₆)haloalkoxy or is (C₄-C₆)cycloalkenyl or is (C₃-C₆)cycloalkyl wherein the last two mentioned groups are unsubstituted or substituted by halogen or by one or two (C₁-C₄)alkyl groups,
- X: is halogen,
(Y)ₙ is n substituents as defined in claim 1, and
- n: is 0, 1, 2, 3, 4 or 5,
are suitable as herbicides or plant growth regulators. The compounds can be prepared according to the process of claim 7 via intermediates known in principle.

## Description

The invention relates to the technical field of crop protection agents, such as herbicides and plant growth regulators, and in particular to herbicides for selective control of harmful plants in crops of useful plants.

It is known that 2-amino-4-(N-phenylalkylamino)-1,3,5-triazines substituted in the 6-position, which may in each case carry further substituents, have herbicidal and plant-growth-regulating properties; cf. WO-A-98/15537, WO-A-97/08156, DE-A-19641691, WO-A-98/34925, WO-A-97/00254 and the literature cited in these references. A subgroup of such compounds with effective herbicidal properties are compounds having cyclic substituents in the α-position to the amino group of the phenylalkylamino radical and are known from WO-A-99/65882 and WO-A-0/56722. It is also known from WO-A-01/44208 that optical isomers of 2,4-amino-1,3,5-triazines may be used as herbicides.

However, the known active compounds are not equally suitable as herbicides for controlling harmful plants in crops of useful plants. For example they may be less active against certain species of harmful plants than required or, under certain circumstances, at doses required to control the harmful plants, they may cause damage to the crop plants. Thus it is an object of the present invention to provide alternative active compounds of the 2,4-diamino-1,3,5-triazine type which, if appropriate, can be employed advantageously as herbicides or plant growth regulators.

Surprisingly, it has been found that certain stereoisomers of the active compounds mentioned have particularly advantageous effects.

The present invention provides compounds of the formula (I) and salts thereof, 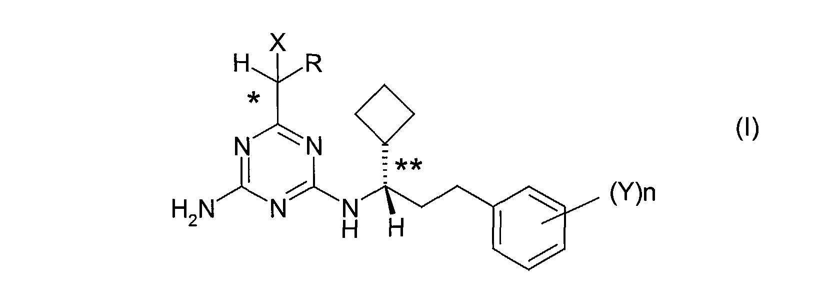 wherein the chiral carbon atom alpha to the amino group of the phenylalkylamino radical (marked ** in formula (I) above) is in the S-configuration, as indicated in formula (I) (i.e. the radicals at the chiral center are ordered NH, cyclobutyl, CH₂, hydrogen, in descending size order according to the Cahn-Ingold-Prelog system) whilst the chiral carbon atom to which the R and X radicals are bonded (marked * in formula (I) above) can be in either the R or S configuration and wherein
- R: is (C₁-C₆)alkyl, (C₁-C₆)haloalkyl, [(C₁-C₆)alkoxy](C₁-C₆)alkyl, (C₂-C₆)alkenyl, (C₂-C₆)haloalkenyl, (C₁-C₆)alkoxy, (C₁-C₆)haloalkoxy or is (C₄-C₆)cycloalkenyl or is (C₃-C₆)cycloalkyl wherein the last two mentioned groups are unsubstituted or substituted by halogen or by one or two (C₁-C₄)alkyl groups;
- X: is halogen,
- (Y)ₙ: is n substituents Y, where Y in each case independently of one another is halogen, hydroxyl, amino, nitro, formyl, carboxyl, cyano, thiocyanato, aminocarbonyl or
(C₁-C₆)alkyl, (C₁-C₆)alkoxy, (C₁-C₆)alkylthio, mono(C₁-C₆)alkylamino, di(C₁-C₆)alkylamino, (C₂-C₆)alkenyl, (C₂-C₆)alkynyl, [(C₁-C₆)alkyl]carbonyl, [(C₁-C₆)alkoxy]carbonyl, mono(C₁-C₆)alkylaminocarbonyl, di(C₁-C₆)alkylaminocarbonyl, N-(C₁-C₆)alkanoylamino or N-(C₁-C₆)alkanoyl-N-(C₁-C₆)alkylamino,
where each of the 13 last-mentioned radicals is unsubstituted or substituted, preferably is unsubstituted or substituted by one or more radicals selected from the group consisting of halogen, hydroxyl, amino, nitro, formyl, carboxyl, cyano, thiocyanato, (C₁-C₄)alkoxy, (C₁-C₄)haloalkoxy, (C₁-C₄)alkylthio, (C₁-C₄)haloalkylthio, mono(C₁-C₄)alkylamino, di(C₁-C₄)alkylamino, (C₃-C₉)cycloalkyl, (C₃-C₉)cycloalkylamino, [(C₁-C₄)alkyl]carbonyl, [(C₁-C₄)alkoxy]carbonyl, aminocarbonyl, mono(C₁-C₄)alkylaminocarbonyl, di(C₁-C₄)alkylaminocarbonyl, phenyl, phenoxy, phenylthio, phenylcarbonyl, heterocyclyl, heterocyclyloxy, heterocyclylthio and heterocyclylamino,
where each of the 8 last-mentioned radicals is unsubstituted or substituted, preferably is unsubstituted or substituted by one or more substituents selected from the group consisting of halogen, nitro, cyano, (C₁-C₄)alkyl, (C₁-C₄)alkoxy, (C₁-C₄)alkylthio, (C₁-C₄)haloalkyl, (C₁-C₄)haloalkoxy, formyl, (C₁-C₄)alkylcarbonyl and (C₁-C₄)alkoxycarbonyl,
or is (C₃-C₉)cycloalkyl, (C₃-C₉)cycloalkoxy, (C₃-C₉)cycloalkylamino, phenyl, phenoxy, phenylthio, phenylcarbonyl, heterocyclyl, heterocyclyloxy, heterocyclylthio or heterocyclylamino,
where each of the 11 last-mentioned radicals is unsubstituted or substituted, preferably is unsubstituted or substituted by one or more radicals selected from the group consisting of halogen, hydroxyl, amino, nitro, formyl, carboxyl, cyano, thiocyanato, (C₁-C₆)alkyl, (C₁-C₆)haloalkyl, (C₁-C₆)alkoxy, (C₁-C₆)haloalkoxy, (C₁-C₆)alkylthio, (C₁-C₆)haloalkylthio, mono(C₁-C₆)alkylamino, di(C₁-C₆)alkylamino, (C₃-C₉)cycloalkyl, [(C₁-C₆)alkyl]carbonyl, [(C₁-C₆)alkoxy]carbonyl, aminocarbonyl, mono(C₁-C₆)alkylaminocarbonyl and di(C₁-C₆)alkylaminocarbonyl,
or two adjacent radicals Y together form a fused-on ring having 4 to 6 ring atoms, the ring being carbocyclic or additonally containing hetero ring atoms from the group consisting of O, S and N and being unsubstituted or substituted by one or more radicals from the group consisting of halogen, (C₁-C₆)alkyl and oxo,
- n: is 0, 1, 2, 3, 4 or 5, preferably 0, 1, 2, or 3.

Where compounds of the formula (I) are capable of forming tautomers whose structure is formally not represented or included in the formula (I), these tautomers are nevertheless embraced by the definition of the compounds of the formula (I) according to the invention.

The compound of formula (I) may be present in mixtures with other stereoisomers thereof, for example in a racemic mixture or other mixture of stereoisomers.

The compounds of formula (I) are preferably used by way of resolved compounds where they are present as a pure stereoisomer with respect to the carbon atom marked (*) in formula (I) which carbon atom is then either in the S configuration or the R configuration, or as a mixture of the R and S stereoisomers, in which one of the stereoisomers is present in excess. The predominant stereoisomer with respect to this chiral carbon atom is preferably present at from 60 to 100%, in particular from 70 to 100%, especially from 80 to 100%, most preferably from 90 to 100%, based on the total amount of R and S isomers with respect to this carbon atom.

Further, the compounds of formula (I) are preferably used by way of resolved compounds where they are present as a pure stereoisomer in which the carbon atom marked (**) in formula (I) is in the S configuration or as a mixture of the R and S stereoisomers, in which the S stereoisomer is present in excess. The S stereoisomer is preferably present at from 60 to 100%, in particular from 70 to 100%, especially from 80 to 100%, most preferably from 90 to 100%, based on the total amount of R and S isomers.

Compounds in which both the chiral carbon atom marked (*) and the chiral carbon atom marked (**) in formula (I) are in the S form are preferred. Mixtures in which the aforementioned compounds are predominant compared to the other stereo isomers are also preferred.

The stereoisomers of compounds of the formula (I) may contain further centers of asymmetry, for example in the radicals R and Y. The compounds of the formula (I) are present as pure enantiomers, or enantiomer mixtures in which one of the enantiomers is present in excess or as racemates. Whether enantiomers are present in excess is measurable, for example, by a specific rotation of the polarized light (optical rotatory dispersion) or by other methods for determining the enantiomeric excess (ee% is the percentage enantiomeric excess), for example by chromatography over chiral separation materials using methods known in the art.

Further centers of asymmetry in the compound of the formula (I) can be asymmetrically substituted carbon atoms or double bonds, which are not specifically mentioned in formula (I). The possible stereoisomers, which are defined by their specific steric form, such as enantiomers, diastereomers and Z & E isomers, are all embraced by the formula (I).

In principle, the stereoisomers can be obtained by customary methods from mixtures of the stereoisomers or can be prepared by stereoselective reactions in combination with the use of stereochemically pure or enriched starting materials. Compounds of the formula (I) which are substantially enantiomerically pure can also be obtained by the separation of racemates by customary methods, for example by crystallisation or chiral chromatography.

Compounds of the formula (I) can form salts by addition of a suitable inorganic or organic acid, such as, for example, HCl, HBr, H₂SO₄ or HNO₃, but also oxalic acid or sulfonic acids, to a basic group, such as, for example, amino or alkylamino. Suitable substituents which can be deprotonated, such as, for example, sulfonic acids or carboxylic acids, can form inert salts with groups which for their part can be protonated, such as amino groups. Salts can also be formed by replacing the hydrogen of suitable substituents, such as, for example, sulfonic acids or carboxylic acids, by an agriculturally suitable cation. These salts are, for example, metal salts, in particular alkali metal salts or alkaline earth metal salts, in particular sodium salts and potassium salts, or else ammonium salts, salts with organic amines or quaternary ammonium salts. Where reference is made to compounds of the formula (I) or compounds (I), that reference should be taken as referring to compounds of the formula (I) and their salts, unless otherwise specifically indicated.

Compounds of the formula (I) in which the sum of carbon atoms in any one non-cyclic radical Y does not exceed 10 are preferred. More preferred are compounds of the formula (I) in which the sum of carbon atoms in any one non-cyclic radical Y does not exceed 6 and most preferred are compounds of the formula (I) in which the sum of carbon atoms in any one non-cyclic radical Y does not exceed 4.

In formula (I) and all the formulae hereinbelow halogen is preferably fluorine, chlorine, bromine or iodine.

In formula (I) and all the formulae hereinbelow, the radicals alkyl, alkoxy, haloalkyl, haloalkoxy, alkylamino and alkylthio and the corresponding unsaturated and/or substituted radicals can in each case be of straight or branched chain. Unless specifically mentioned, the lower carbon skeletons are preferred. These preferred lower skeletons have, for example 1 to 6 carbon atoms in the case of saturated radicals or 2 to 6 carbon atoms in the case of unsaturated radicals. Alkyl radicals, and alkyl radicals in composite radicals (such as alkoxy, haloalkyl, and the like) that lie within the preferred ranges are, for example, methyl, ethyl, n- or isopropyl, n-, iso-, tert- or 2-butyl, pentyls, hexyls, such as n-hexyl, isohexyl and 1,3-dimethylbutyl; likewise alkenyl or alkynyl radicals means those possible unsaturated radicals which correspond to the above mentioned alkyl radicals; thus alkenyl is, for example, allyl, 1-methylprop-2-en-1-yl, 2-methylprop-2-en-1-yl, but-2-en-1-yl, but-3-en-1-yl, 1-methylbut-3-en-1-yl and 1-methylbut-2-en-1-yl; alkynyl is, for example, propargyl, but-2-yn-1-yl, but-3-yn-1-yl, 1-methylbut-3-yn-1-yl.

Of these lower radicals, those having 1 to 4 carbon atoms for saturated radicals and 2 to 4 carbon atoms in the case of unsaturated radicals are more preferred. Those radicals having one or two carbon atoms are particularly preferred.

In general, preference is given to substituents selected from the group consisting of halogen, for example fluorine and chlorine, (C₁-C₄)alkyl, preferably methyl or ethyl, (C₁-C₄)haloalkyl, preferably trifluoromethyl, (C₁-C₄)alkoxy, preferably methoxy or ethoxy, (C₁-C₄)haloalkoxy, nitro and cyano. Particular preference is given here to the substituents methyl, methoxy, fluorine and chlorine.

Alkylidene, is the radical of a straight-chain or branched alkane which is attached via a double bond to the rest of the molecule. In the case of a branched alkane, the only possible positions are those where two hydrogen atoms can be replaced by the double bond; examples of alkylidene radicals are =CH₂, =CH-CH₃, =C(CH₃)-CH₃, =C(CH₃)-C₂H₅ or =C(C₂H₅)-C₂H₅.
Cycloalkyl is a carbocyclic saturated ring system having preferably 3-8 carbon atoms, for example cyclopropyl, cyclobutyl, cyclopentyl or cyclohexyl. Substituted cycloalkyl includes cyclic systems where the substituents are attached to the alkyl radical via a double bond, for example an alkylidene group such as methylidene. Substituted cycloalkyl also includes polycyclic aliphatic systems, such as, for example, bicyclo[1.1.0]butan-1-yl, bicyclo[1.1.0]butan-2-yl, bicyclo[2.1.0]pentan-1-yl, bicyclo[2.1.0]pentan-2-yl, bicyclo[2.1.0]pentan-5-yl, adamantan-1-yl and adamantan-2-yl.
Cycloalkenyl is a carbocyclic nonaromatic, partially unsaturated ring system having preferably 4-8 carbon atoms, for example 1-cyclobutenyl, 2-cyclobutenyl, 1-cyclopentenyl, 2-cyclopentenyl, 3-cyclopentenyl, or 1-cyclohexenyl, 2-cyclohexenyl, 3-cyclohexenyl, 1,3-cyclohexadienyl or 1,4-cyclohexadienyl. For substituted cycloalkenyl, the illustrations for substituted cycloalkyl apply correspondingly.
Haloalkyl, -alkenyl and -alkynyl are alkyl, alkenyl and alkynyl radicals, respectively, which are partially or fully substituted by one or more species of halogen, the halogen being preferably fluorine, chlorine or bromine, in particular by fluorine or chlorine, for example monohaloalkyl, perhaloalkyl, CF₃, CHF₂, CH₂F, CF₃CF₂, CH₂FCHCl, CCl₃, CHCl₂, CH₂CH₂Cl; haloalkoxy is, for example, OCF₃, OCHF₂, OCH₂F, CF₃CF₂O, OCH₂CF₃ and OCH₂CH₂Cl; this applies correspondingly to haloalkenyl and other halogen-substituted radicals.
Halocycloalkyl and halocycloakenyl are respectively cycloalkyl and cycloalkenyl radicals which are partially or fully substituted by one or more species of halogen atom, selected from fluorine, chlorine, bromine and iodine, and particularly from fluorine and chlorine.

Aryl is a mono-, bi- or polycyclic aromatic system, for example phenyl, naphthyl, tetrahydronaphthyl, indenyl, indanyl, pentalenyl, fluorenyl and the like, preferably phenyl.
A heterocyclic radical or ring (heterocyclyl) is preferably unless otherwise defined, independently of one another in each case, a heterocyclic radical having 3 to 7 ring atoms and 1 or more hetero atoms from the group consisting of N, O and S.
The heterocyclyl group can be saturated, unsaturated or heteroaromatic; unless defined otherwise, it preferably contains one or more, in particular 1, 2 or 3, heteroatoms in the ring, selected from the group consisting of N, O and S; preferably it is an aliphatic heterocyclyl radical having 3 to 7 ring atoms or a heteroaromatic radical having 5 or 6 ring atoms. The heterocyclic radical can, for example, be a heteroaromatic radical or ring (heteroaryl), such as, for example, a mono-, bi- or polycyclic aromatic system, in which at least 1 ring contains one or more heteroatoms.

Preferred are heteroaromatic rings having one heteroatom selected from the group consisting of N, O and S, for example pyridyl, pyrrolyl, thienyl or furyl; also preferred are the corresponding heteroaromatic rings having 2 or 3 heteroatoms, for example pyrimidinyl, pyridazinyl, pyrazinyl, triazinyl, thiazolyl, thiadiazolyl, oxazolyl, isoxazolyl, pyrazolyl, imidazolyl and triazolyl.
Also preferred are partially or fully hydrogenated heterocyclic radicals having one heteroatom selected from the group consisting of N, O and S, for example oxiranyl, oxetanyl, oxolanyl (= tetrahydrofuryl), oxanyl, pyrrolidyl or piperidyl. Also preferred are partially or fully hydrogenated heterocyclic radicals having 2 heteroatoms selected from the group consisting of N, O and S, for example piperazinyl, dioxolanyl, oxazolinyl, isoxazolinyl, oxazolidinyl, isoxazolidinyl and morpholinyl.

Heterocyclyl is particularly preferably a heterocyclic radical having 5 or 6 ring atoms and 2 or 3 heteroatoms from the group consisting of N, O and S, in particular pyrimidinyl, pyridazinyl, pyrazinyl, triazinyl, thiazolyl, thiadiazolyl, oxazolyl, isoxazolyl, pyrazolyl, imidazolyl, triazolyl or piperazinyl, dioxolanyl, oxazolinyl, isoxazolinyl, oxazolidinyl, isoxazolidinyl or morpholinyl.

Possible substituents for a substituted heterocyclic radical are the substituents mentioned further below, and additionally also oxo. The oxo group can be present at the hetero ring atoms which can exist in different oxidation states, for example at N and S, and/or at the ring carbon atoms

Substituted radicals, such as a substituted alkyl, cycloalkyl, alkenyl, cycloalkyl, alkynyl, aryl, phenyl, benzyl, heterocyclyl and heteroaryl radicals are, for example, a substituted radical derived from the unsubstituted skeleton, where the substituents are, for example, one or more, preferably 1, 2 or 3, radicals selected from the group consisting of halogen, alkoxy, haloalkoxy, alkylthio, cycloalkyl, hydroxyl, amino, nitro, thiocyanato, carboxyl, cyano, azido, alkoxy-carbonyl, alkyl-carbonyl, formyl, carbamoyl, mono- and dialkylaminocarbonyl, substituted amino, such as acylamino, mono- and dialkylamino, and alkylsulfinyl, haloalkylsulfinyl, alkylsulfonyl, haloalkylsulfonyl and, in the case of cyclic radicals, also alkyl, haloalkyl, alkylthioalkyl, alkoxyalkyl, optionally substituted mono- and dialkylaminoalkyl and hydroxylalkyl; the term "substituted radicals", such as substituted alkyl and the like, also includes as substituents, in addition to the saturated hydrocarbon-containing radicals mentioned, corresponding unsaturated aliphatic and aromatic radicals, such as unsubstituted or substituted alkenyl, alkynyl, alkenyloxy, alkynyloxy, phenyl, phenoxy etc. Substituted cyclic radicals having aliphatic moieties in the ring include cyclic systems having substituents which are attached to the ring via a double bond, for example cyclic systems substituted by an alkylidene group, such as methylidene or ethylidene. The preferred substituents for the above groups also apply where those groups appear as part of a complex radical such as, for example, alkylthio, alkoxy, alkylcarbonyl, alkylaminocarbonyl, phenoxy, phenylthio, heterocyclyloxy

Substituted amino, such as mono- or disubstituted amino, is an N-substituted amino radical, substituted by, for example, one or two identical or different radicals selected from the group consisting of alkyl, cycloalkyl, alkoxy, acyl and aryl; preferably mono- and dialkylamino, mono- and diarylamino, acylamino, N-alkyl-N-arylamino, N-alkyl-N-acylamino and nitrogen-containing heterocycles attached via a nitrogen atom; preference is given to alkyl radicals having 1 to 4 carbon atoms; aryl is preferably phenyl or substituted phenyl; acyl is as defined below but is preferably (C₁-C₄)alkanoyl. The definition of substituents possible for substituted amino applies correspondingly to those for substituted hydroxylamino or hydrazino radicals.

Unsubstituted or substituted phenyl is phenyl which is unsubstituted or substituted by one or more radicals, preferably it is mono, di or trisubstituted, by identical or different radicals selected from the group consisting of halogen, (C₁-C₄)alkyl, (C₁-C₄)alkoxy, (C₁-C₄)haloalkyl, (C₁-C₄)haloalkoxy and nitro, for example o-, m- and p-tolyl, dimethylphenyls, 2-, 3- and 4-chlorophenyl, 2-,3- and 4-fluorophenyl, 2-, 3-and 4-trifluoromethylphenyl, 2,4-, 3,5-, 2,5- and 2,3-dichlorophenyl and - difluorophenyl, 2,3,4-trifluoro- and -trichlorophenyl, o-, m- and p-methoxyphenyl.

Acyl is a radical of an organic acid which is formally formed by removing a hydroxyl group from the acid function, where the organic radical in the acid can also be attached to the acid function via a heteroatom. Examples of acyl are the radical - CO-R of a carboxylic acid HO-CO-R and radicals of acids derived therefrom, such as thiocarbonic acid, unsubstituted or N-substituted iminocarboxylic acids or the radical of carbonic monoesters, N-substituted carbamic acids, sulfonic acids, sulfinic acids, N-substituted sulfonamide acids, phosphonic acids, phosphinic acids. Acyl is, for example, formyl, alkyl-carbonyl, such as [(C₁-C₄)alkyl]carbonyl, phenylcarbonyl, alkyloxycarbonyl, phenyloxycarbonyl, benzyloxycarbonyl, alkylsulfonyl, alkylsulfinyl, N-alkylalkylimidoyl and other radicals of organic acids. The radicals can in each case be further substituted in the alkyl or phenyl moiety, for example in the alkyl moiety by one or more radicals selected from the group consisting of halogen, alkoxy, phenyl and phenoxy; examples of substituents in the phenyl moiety have already been mentioned for substituted phenyl.

Acyl is preferably an acyl radical in the more restricted sense, i.e. a radical of an organic acid where the acid group is directly attached to the carbon atom of an organic radical, for example formyl, alkyl-carbonyl, such as [(C₁-C₄)alkyl]carbonyl, more specifically acetyl, or phenylcarbonyl, alkylsulfonyl, alkylsulfinyl and other radicals of organic acids.

In particular for reasons of better herbicidal activity, better selectivity and/or easier preparation, the compounds of the formula (I) or their salts of particular interest are those in which individual radicals have one of the preferred meanings already mentioned or mentioned hereinbelow, or, in particular, those in which one or more of the preferred meanings already mentioned or mentioned hereinbelow are combined.

Thus preferred compounds (I) are those in which
- R: is (C₁-C₆)alkyl, (C₁-C₆)haloalkyl, [(C₁-C₆)alkoxy](C₁-C₆)alkyl, (C₂-C₆)alkenyl, (C₂-C₆)haloalkenyl, (C₁-C₆)alkoxy, (C₁-C₆)haloalkoxy or is (C₄-C₆)cycloalkenyl or is (C₃-C₆)cycloalkyl wherein the last two mentioned groups are unsubstituted or substituted by halogen, preferably
- R: is (C₁-C₄)alkyl, (C₁-C₄)haloalkyl, [(C₁-C₄)alkoxy](C₁-C₄)alkyl, (C₂-C₄)alkenyl, (C₂-C₄)haloalkenyl, (C₁-C₄)alkoxy, (C₁-C₄)haloalkoxy or is (C₄-C₆)cycloalkenyl or is (C₃-C₆)cycloalkyl wherein the last two mentioned groups are unsubstituted or substituted by halogen.

Also preferred are compounds (I) in which;
- R: is (C₁-C₆)alkyl, (C₁-C₆)haloalkyl, (C₂-C₆)alkenyl or
(C₂-C₆)haloalkenyl, preferably
- R: is (C₁-C₄)alkyl, (C₁-C₄)haloalkyl, (C₂-C₄)alkenyl or
(C₂-C₄)haloalkenyl.

Also preferred are compounds (I) in which
- R: is (C₁-C₆)alkyl, in particular (C₁-C₄)alkyl

The most preferred compounds (I) are those in which R is methyl, ethyl or propyl.

Compounds (I) in which X is fluorine or chlorine or bromine are preferred. More preferred are compounds (I) in which X is fluorine or chlorine and most preferred are compounds (I) in which X is fluorine.

Preferred are compounds (I) in which:
- (Y)ₙ: is n substituents Y, where Y in each case independently of one another is halogen, hydroxyl, amino, nitro, formyl, carboxyl, cyano, thiocyanato, aminocarbonyl or
(C₁-C₆)alkyl, (C₁-C₆)alkoxy, (C₁-C₆)alkylthio, mono(C₁-C₆)alkylamino, di(C₁-C₆)alkylamino, (C₂-C₆)alkenyl, (C₂-C₆)alkynyl, [(C₁-C₆)alkyl]carbonyl, [(C₁-C₆)alkoxy]carbonyl, mono(C₁-C₆)alkylaminocarbonyl, di(C₁-C₆)alkylaminocarbonyl, N-(C₁-C₆)alkanoylamino or N-(C₁-C₆)alkanoyl-N-(C₁-C₆)alkylamino,
where each of the 13 last-mentioned radicals is unsubstituted or substituted, preferably is unsubstituted or substituted by one or more radicals selected from the group consisting of halogen, hydroxyl, amino, nitro, formyl, carboxyl, cyano, thiocyanato, (C₁-C₄)alkoxy, (C₁-C₄)haloalkoxy and (C₁-C₄)alkylthio,
or is (C₃-C₉)cycloalkyl, (C₃-C₉)cycloalkoxy, (C₃-C₉)cycloalkylamino, phenyl, phenoxy, phenylthio, phenylcarbonyl, heterocyclyl, heterocyclyloxy, heterocyclylthio or heterocyclylamino,
where each of the 11 last-mentioned radicals is unsubstituted or substituted, preferably is unsubstituted or substituted by one or more radicals selected from the group consisting of halogen, hydroxyl, amino, nitro, formyl, carboxyl, cyano, thiocyanato, (C₁-C₆)alkyl, (C₁-C₆)haloalkyl, (C₁-C₆)alkoxy, (C₁-C₆)haloalkoxy and (C₁-C₆)alkylthio
or two adjacent radicals Y together are a fused-on ring having 4 to 6 ring atoms, the ring being carbocyclic or containing hetero ring atoms from the group consisting of O, S and N and being unsubstituted or substituted by one or more radicals from the group consisting of halogen, (C₁-C₆)alkyl and oxo.

More preferred are compounds (I) in which;
- (Y)ₙ: is n substituents Y, where Y in each case independently of one another is preferably halogen, hydroxyl, amino, nitro, formyl, carboxyl, cyano, thiocyanato, aminocarbonyl or
(C₁-C₆)alkyl, (C₁-C₆)alkoxy, (C₁-C₆)alkylthio, mono(C₁-C₆)alkylamino, di(C₁-C₆)alkylamino, (C₂-C₆)alkenyl, (C₂-C₆)alkynyl, [(C₁-C₆)alkyl]carbonyl, [(C₁-C₆)alkoxy]carbonyl, mono(C₁-C₆)alkyl-aminocarbonyl, di(C₁-C₆)alkylaminocarbonyl, N-(C₁-C₆)alkanoylamino or N-(C₁-C₆)alkanoyl-N-(C₁-C₆)alkylamino,
where each of the 13 last-mentioned radicals is unsubstituted or substituted preferably is unsubstituted or substituted by one or more radicals selected from the group consisting of halogen, hydroxyl, amino, nitro, formyl, carboxyl, cyano, thiocyanato, (C₁-C₄)alkoxy, (C₁-C₄)haloalkoxy and (C₁-C₄)alkylthio
or is (C₃-C₉)cycloalkyl.

Still more preferred are compounds (I) in which
- (Y)ₙ: is n substituents Y, where Y in each case independently of one another is preferably halogen, hydroxyl, amino, nitro, formyl, carboxyl, cyano, thiocyanato, aminocarbonyl or
(C₁-C₆)alkyl, (C₁-C₆)alkoxy, (C₁-C₆)alkylthio, mono(C₁-C₆)alkylamino, di(C₁-C₆)alkylamino, (C₂-C₆)alkenyl, (C₂-C₆)alkynyl, [(C₁-C₆)alkyl]carbonyl, [(C₁-C₆)alkoxy]carbonyl, mono(C₁-C₆)alkyl-aminocarbonyl, di(C₁-C₆)alkylaminocarbonyl, N-(C₁-C₆)alkanoylamino or N-(C₁-C₆)alkanoyl-N-(C₁-C₆)alkylamino,
where each of the 13 last mentioned radicals is unsubstituted or
substituted and is preferably unsubstituted or substituted by halogen,
or is (C₃-C₉)cycloalkyl.

Yet more preferred are compounds (I) in which;
- (Y)ₙ: is n substituents Y, where Y in each case independently of one another is preferably halogen, hydroxyl, nitro, cyano, (C₁-C₆)alkyl, (C₁-C₆)alkoxy or (C₁-C₆)alkylthio,
where each of the 3 last mentioned radicals is unsubstituted or
substituted and is preferably unsubstituted or substituted by halogen,
or is (C₃-C₉)cycloalkyl.

Yet further preferred are those compounds (I) in which;
- (Y)ₙ: is n substituents Y, where Y in each case independently of one another is preferably halogen, hydroxyl, nitro, cyano, (C₁-C₆)alkyl, (C₁-C₆)alkoxy or (C₁-C₆)alkylthio,
where each of the 3 last mentioned radicals is unsubstituted or
substituted and is preferably unsubstituted or substituted by halogen.

Compounds (I) in which n is 1, 2 or 3 are preferred.

Also preferred are compounds (I) in which:
- R: is (C₁-C₆)alkyl, (C₁-C₆)haloalkyl, [(C₁-C₆)alkoxy](C₁-C₆)alkyl, (C₂-C₆)alkenyl, (C₂-C₆)haloalkenyl, (C₁-C₆)alkoxy, (C₁-C₆)haloalkoxy or is (C₄-C₆)cycloalkenyl or is (C₃-C₆)cycloalkyl wherein the last two mentioned groups are unsubstituted or substituted by halogen or by one or two (C₁-C₄)alkyl groups; preferably
R is (C₁-C₄)alkyl, (C₁-C₄)haloalkyl, [(C₁-C₄)alkoxy](C₁-C₄)alkyl, (C₂-C₄)alkenyl, (C₂-C₄)haloalkenyl, (C₁-C₄)alkoxy, (C₁-C₄)haloalkoxy or is (C₄-C₆)cycloalkenyl or is (C₃-C₆)cycloalkyl wherein the last two mentioned groups are unsubstituted or substituted by halogen or by one or two (C₁-C₄)alkyl groups, and
- X: is halogen, and
- (Y)ₙ: is n substituents Y, where Y in each case independently of one another is halogen, hydroxyl, amino, nitro, formyl, carboxyl, cyano, thiocyanato, aminocarbonyl or
(C₁-C₆)alkyl, (C₁-C₆)alkoxy, (C₁-C₆)alkylthio, mono(C₁-C₆)alkylamino, di(C₁-C₆)alkylamino, (C₂-C₆)alkenyl, (C₂-C₆)alkynyl, [(C₁-C₆)alkyl]carbonyl, [(C₁-C₆)alkoxy]carbonyl, mono(C₁-C₆)alkylaminocarbonyl, di(C₁-C₆)alkylaminocarbonyl, N-(C₁-C₆)alkanoylamino or N-(C₁-C₆)alkanoyl-N-(C₁-C₆)alkylamino,
where each of the 13 last-mentioned radicals is unsubstituted or substituted, preferably is unsubstituted or substituted by one or more radicals selected from the group consisting of halogen, hydroxyl, amino, nitro, formyl, carboxyl, cyano, thiocyanato, (C₁-C₄)alkoxy, (C₁-C₄)haloalkoxy and (C₁-C₄)alkylthio,
or is (C₃-C₉)cycloalkyl, (C₃-C₉)cycloalkoxy, (C₃-C₉)cycloalkylamino, phenyl, phenoxy, phenylthio, phenylcarbonyl, heterocyclyl, heterocyclyloxy, heterocyclylthio or heterocyclylamino,
where each of the 11 last-mentioned radicals is unsubstituted or substituted, preferably is unsubstituted or substituted by one or more radicals selected from the group consisting of halogen, hydroxyl, amino, nitro, formyl, carboxyl, cyano, thiocyanato, (C₁-C₆)alkyl, (C₁-C₆)haloalkyl, (C₁-C₆)alkoxy, (C₁-C₆)haloalkoxy and (C₁-C₆)alkylthio
or two adjacent radicals Y together are a fused-on ring having 4 to 6 ring atoms, the ring being carbocyclic or containing hetero ring atoms from the group consisting of O, S and N and being unsubstituted or substituted by one or more radicals from the group consisting of halogen, (C₁-C₆)alkyl and oxo and,
- n: is 0, 1, 2, 3, 4 or 5.

More preferred are compounds (I) in which:
- R: is (C₁-C₆)alkyl, (C₁-C₆)haloalkyl, (C₂-C₆)alkenyl or
(C₂-C₆)haloalkenyl, preferably
R is (C₁-C₄)alkyl, (C₁-C₄)haloalkyl, (C₂-C₄)alkenyl or (C₂-C₄)haloalkenyl, and
- X: is fluorine or chlorine or bromine, and
- (Y)ₙ: is n substituents Y, where Y in each case independently of one another is preferably halogen, hydroxyl, amino, nitro, formyl, carboxyl, cyano, thiocyanato, aminocarbonyl or
(C₁-C₆)alkyl, (C₁-C₆)alkoxy, (C₁-C₆)alkylthio, mono(C₁-C₆)alkylamino, di(C₁-C₆)alkylamino, (C₂-C₆)alkenyl, (C₂-C₆)alkynyl, [(C₁-C₆)alkyl]carbonyl, [( C₁-C₆)alkoxy]carbonyl, mono(C₁-C₆)alkyl-aminocarbonyl, di(C₁-C₆)alkylaminocarbonyl, N-(C₁-C₆)alkanoylamino or N-(C₁-C₆)alkanoyl-N-(C₁-C₆)alkylamino,
where each of the 13 last-mentioned radicals is unsubstituted or substituted preferably is unsubstituted or substituted by one or more radicals selected from the group consisting of halogen, hydroxyl, amino, nitro, formyl, carboxyl, cyano, thiocyanato, (C₁-C₄)alkoxy, (C₁-C₄)haloalkoxy and (C₁-C₄)alkylthio
or is (C₃-C₉)cycloalkyl, and
- n: is 0, 1, 2, 3, 4 or 5.

More preferred still are compounds (I) in which:
- R: is (C₁-C₆)alkyl, (C₁-C₆)haloalkyl, (C₂-C₆)alkenyl or
(C₂-C₆)haloalkenyl, preferably
R is (C₁-C₄)alkyl, (C₁-C₄)haloalkyl, (C₂-C₄)alkenyl or (C₂-C₄)haloalkenyl and
- X: is fluorine or chlorine, and
- (Y)ₙ: is n substituents Y, where Y in each case independently of one another is preferably halogen, hydroxyl, amino, nitro, formyl, carboxyl, cyano, thiocyanato, aminocarbonyl or
(C₁-C₆)alkyl, (C₁-C₆)alkoxy, (C₁-C₆)alkylthio, mono(C₁-C₆)alkylamino, di(C₁-C₆)alkylamino, (C₂-C₆)alkenyl, (C₂-C₆)alkynyl, [(C₁-C₆)alkyl]carbonyl, [(C₁-C₆)alkoxy]carbonyl, mono(C₁-C₆)alkyl-aminocarbonyl, di(C₁-C₆)alkylaminocarbonyl, N-(C₁-C₆)alkanoylamino or N-(C₁-C₆)alkanoyl-N-(C₁-C₆)alkylamino,
where each of the 13 last mentioned radicals is unsubstituted or
substituted and is preferably unsubstituted or substituted by halogen,
or is (C₃-C₉)cycloalkyl and
- n: is 0, 1, 2, 3, 4 or 5.

Still more preferred compounds (I) are those in which:
- R: is (C₁-C₆)alkyl, preferably (C₁-C₄)alkyl and
- X: is fluorine or chlorine, and
- (Y)ₙ: is n substituents Y where Y in each case independently of one another is preferably halogen, hydroxyl, nitro, cyano, (C₁-C₆)alkyl, (C₁-C₆)alkoxy or (C₁-C₆)alkylthio,
where each of the 3 last mentioned radicals is unsubstituted or
substituted and is preferably unsubstituted or substituted by halogen,
or is (C₃-C₉)cycloalkyl, and
- n: is 0, 1, 2,or 3.

The most preferred compounds (I) are those in which:
- R: is (C₁-C₆)alkyl, preferably (C₁-C₄)alkyl and most preferably methyl, ethyl or propyl, and
- X: is fluorine, and
- (Y)ₙ: is n substituents Y, where Y in each case independently of one another is preferably halogen, hydroxyl, nitro, cyano, (C₁-C₆)alkyl, (C₁-C₆)alkoxy or (C₁-C₆)alkylthio,
where each of the 3 last mentioned radicals is unsubstituted or
substituted and is preferably unsubstituted or substituted by halogen
and
- n: is 0,1,2, or 3.

Preferred are also compounds of the formula (I) in which two or more of the defined preferred features are combined. Also preferred are compounds in which one or more radicals are identical to those of the examples of the Tables 1 to 6 further below or are one or more radicals which fall within generic groups, preferably preferred generic groups, corresponding to those radicals of the examples of the Tables 1 to 6 further below. Also preferred are compounds or combinations of compounds as defined by formulae (Ia) to (If) as shown in the headings of Tables 1 to 6, wherein the general radicals of the formula are defined as in formula (I) above or according to the preferred definitions set forth above.

Particularly preferred compounds (I) are:
*N*-{4-amino-6-[(1*S*)-1-fluoroethyl]-1,3,5-triazin-2-yl}-*N*-[(1*S*)-1-cyclobutyl-3-phenylpropyl]amine.
*N*-{4-amino-6-[(1*S*)-1-fluoroethyl]-1,3,5-triazin-2-yl}-*N*-[(1*S*)-1-cyclobutyl-3-(2-fluorophenyl)propyl]amine.
*N*-{4-amino-6-[(1*S*)-1-fluoroethyl]-1,3,5-triazin-2-yl}-*N*-[(1*S*)-1-cyclobutyl-3-(4-methoxyphenyl)propyl]amine.
*N*-{4-amino-6-[(1*S*)-1-fluoroethyl]-1,3,5-triazin-2-yl}-*N*-[(1*S*)-1-cyclobutyl-3-(4-fluorophenyl)propyl]amine.
*N*-{4-amino-6-[(1*S*)-1-fluoroethyl]-1,3,5-triazin-2-yl}-*N*-[(1*S*)-1-cyclobutyl-3-(4-bromophenyl)propyl]amine.
*N*-{4-amino-6-[(1*S*)-1-fluoroethyl]-1,3,5-triazin-2-yl}-*N*-[(1*S*)-1-cyclobutyl-3-(4-chlorophenyl)propyl]amine.
*N*-{4-amino-6-[(1*S*)-1-fluoropropyl]-1,3,5-triazin-2-yl}-*N*-[(1*S*)-1-cyclobutyl-3-phenylpropyl]amine.
*N*-{4-amino-6-[(1*S*)-1-fluoropropyl]-1,3,5-triazin-2-yl}-*N*-[(1*S*)-1-cyclobutyl-3-(4-fluorophenyl)propyl]amine.
*N*-{4-amino-6-[(1*S*)-1-fluoropropyl]-1,3,5-triazin-2-yl}-*N*-[(1*S*)-1-cyclobutyl-3-(3,4-difluorophenyl)propyl]amine.
*N*-{4-amino-6-[(1*S*)-1-fluoropropyl]-1,3,5-triazin-2-yl}-*N*-[(1*S*)-1-cyclobutyl-3-(3-fluorophenyl)propyl]amine.
*N*-{4-amino-6-[(1*S*)-1-fluoropropyl]-1,3,5-triazin-2-yl}-*N*-[(1*S*)-1-cyclobutyl-3-(3-methoxyphenyl)propyl]amine.
*N*-{4-amino-6-[(1*S*)-1-fluorobutyl]-1,3,5-triazin-2-yl}-*N*-[(1*S*)-1-cyclobutyl-3-phenylpropyl]amine.
*N*-{4-amino-6-[(1*S*)-1-fluorobutyl]-1,3,5-triazin-2-yl}-*N*-[(1*S*)-1-cyclobutyl-3-(2-fluorophenyl)propyl]amine.

Also preferred are the following racemic mixtures of the preferred compounds:
*N*-{4-amino-6-[(1R,*S*)-1-fluoroethyl]-1,3,5-triazin-2-yl}-*N*-[(1*S*)-1-cyclobutyl-3-phenylpropyl]amine.
*N*-{4-amino-6-[(1R,*S*)-1-fluoroethyl]-1,3,5-triazin-2-yl}-*N*-[(1*S*)-1-cyclobutyl-3-(2-fluorophenyl)propyl]amine.
*N*-{4-amino-6-[(R,*S*)-1-fluoroethyl]-1,3,5-triazin-2-yl}-*N*-[(1*S*)-1-cyclobutyl-3-(4-methoxyphenyl)propyl]amine.
*N*-{4-amino-6-[(1R,*S*)-1-fluoroethyl]-1,3,5-triazin-2-yl}-*N*-[(1S)-1-cyclobutyl-3-(4-fluorophenyl)propyl]amine.
*N*-{4-amino-6-[(1R,*S*)-1-fluoroethyl]-1,3,5-triazin-2-yl}-*N*-[(1*S*)-1-cyclobutyl-3-(4-bromophenyl)propyl]amine.
*N*-{4-amino-6-[(1R,*S*)-1-fluoroethyl]-1,3,5-triazin-2-yl}-*N*-[(1*S*)-1-cyclobutyl-3-(4-chlorophenyl)propyl]amine.
*N*-{4-amino-6-[(1R,*S*)-1-fluoropropyl]-1,3,5-triazin-2-yl}-*N*-[(1*S*)-1-cyclobutyl-3-phenylpropyl]amine.
*N*-{4-amino-6-[(1R,*S*)-1-fluoropropyl]-1,3,5-triazin-2-yl}-*N*-[(1*S*)-1-cyclobutyl-3-(4-fluorophenyl)propyl]amine.
*N*-{4-amino-6-[(1R,*S*)-1-fluoropropyl]-1,3,5-triazin-2-yl}-*N*-[(1*S*)-1-cyclobutyl-3-(3,4-difluorophenyl)propyl]amine.
*N*-{4-amino-6-[(1R,*S*)-1-fluoropropyl]-1,3,5-triazin-2-yl}-*N*-[(1*S*)-1-cyclobutyl-3-(3-fluorophenyl)propyl]amine.
*N*-{4-amino-6-[(1R,*S*)-1-fluoropropyl]-1,3,5-triazin-2-yl}-*N*-[(1S)-1-cyclobutyl-3-(3-methoxyphenyl)propyl]amine.
*N*-{4-amino-6-[(1R,*S*)-1-fluorobutyl]-1,3,5-triazin-2-yl}-*N*-[(1*S*)-1-cyclobutyl-3-phenylpropyl]amine.
*N*-{4-amino-6-[(1R,*S*)-1-fluorobutyl]-1,3,5-triazin-2-yl}-*N*-[(1*S*)-1-cyclobutyl-3-(2-fluorophenyl)propyl]amine.

The invention also provides processes for preparing the compounds of the formula (I) or salts thereof, which comprise
a) reacting an optically active compound of the formula (II), 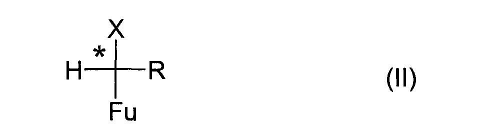 in which Fu is a functional group selected from the group consisting of carboxylic esters, carboxylic orthoesters, carbonyl chlorides, carboxamides, carboxylic anhydrides and trichloromethyl,
   with a compound of the formula (III) or an acid addition salt thereof 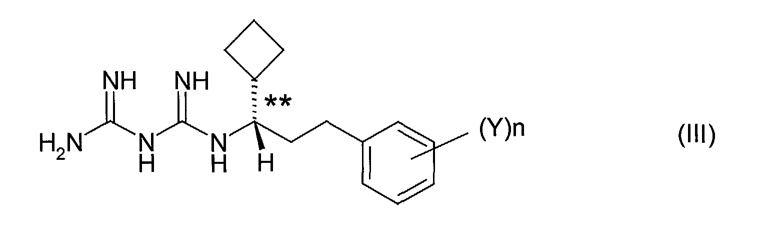 or
b) reacting a compound of the formula (IV), 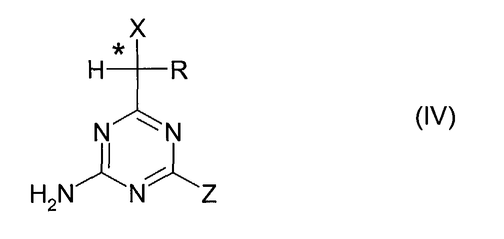 in which Z is an exchangeable radical or a leaving group, for example chlorine, trichloromethyl, (C₁-C₄)alkylsulfonyl and unsubstituted or substituted phenyl-(C₁-C₄)alkylsulfonyl or (C₁-C₄)alkyl-phenylsulfonyl,
   with an amine of the formula (V) or an acid addition salt thereof, 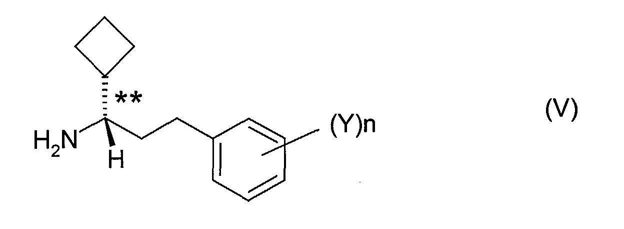 where in the formulae (II), (III), (IV) and (V) of the process variants a) and b) the radicals R, X and Y, the integer n and the configuration of the carbon atoms marked in the formulae with (*) or (**), are as defined in formula (I), or
c) reacting compounds of the formula (II) with compounds of formula (III), or compounds of the formula (IV) with compounds of the formula (V), wherein either or both of the reactants is used in the form of a mixture of enantiomers with respect to the carbon atoms (*) or (**) that is different from that required in the final compound of the formula (I), and then separating the resulting mixture of products to yield a compound of the formula (I) in which the chiral carbon atoms (*) and/or (**) are in the desired form.

The compounds of the formulae (II) and (III) are preferably reacted with base catalysis in an inert organic solvent, such as, for example, tetrahydrofuran (THF), dioxane, acetonitrile, dimethylformamide (DMF), methanol and ethanol, at temperatures between -10 °C and the boiling point of the solvent, preferably at from 20 °C to 60 °C; if acid addition salts of the formula (III) are used, these are generally liberated in situ with the aid of a base. Suitable bases or basic catalysts are alkali metal hydroxides, alkali metal hydrides, alkali metal carbonates, alkali metal alkoxides, alkaline earth metal hydroxides, alkaline earth metal hydrides, alkaline earth metal carbonates or organic bases, such as triethylamine or 1,8-diazabicyclo[5.4.0]undec-7-ene (DBU). The base in question is employed, for example, in the range from 0.1 to 3 molar equivalents, based on the compound of the formula (III). Based on the compound of the formula (III), the compound of the formula (II) can be employed, for example, in equimolar amounts or in an excess of up to 2 molar equivalents. In principle, the corresponding processes are known from the literature (compare: Comprehensive Heterocyclic Chemistry, A.R. Katritzky, C.W. Rees, Pergamon Press, Oxford, New York, 1984, Vol. 3; Part 2B; ISBN 0-08-030703-5, p. 290).

The compounds of the formulae (IV) and (V) are preferably reacted with base catalysis in an inert solvent, such as, for example, THF, dioxane, acetonitrile, DMF, methanol and ethanol, at temperatures between -10 °C and the boiling point of the solvent or solvent mixture in question, preferably at from 20 °C to 60 °C, where the compound (V), if it is employed as acid addition salt, is, if appropriate, liberated in situ using a base. Suitable bases or basic catalysts are alkali metal hydroxides, alkali metal hydrides, alkali metal carbonates, alkali metal alkoxides, alkaline earth metal hydroxides, alkaline earth metal hydrides, alkaline earth metal carbonates or organic bases, such as triethylamine or 1,8-diazabicyclo[5.4.0]undec-7-ene (DBU). The base in question is generally employed in the range from 1 to 3 molar equivalents, based on the compound of formula (IV), and the compound of the formula (IV) can be employed, for example, in equimolar amounts or in an excess up to 2 molar equivalents, based on the compound of the formula (V). In principle, the corresponding processes are known from the literature (cf. Comprehensive Heterocyclic Chemistry, A.R. Katritzky, C.W. Rees, Pergamon Press, Oxford, New York, 1984, Vol. 3; Part 2B; ISBN 0-08-030703-5, p. 482).

The starting materials of the formulae (II), (III), (IV) and (V) are either commercially available, or they can be prepared by, or analogously to, processes known from the literature. The compounds can also be prepared, for example, by one of the processes described below.
Optically active biguanides of the formula (III) can be obtained, for example, by reacting optically active amines of the formula (V) mentioned and cyanoguanidine of the formula H₂N-C(=NH)-NH-CN (see, for example, B. EP-A-492615). In general, the reaction can be carried out effectively under acid catalysis and in the presence of an organic solvent, such as an optionally halogenated hydrocarbon. Suitable catalysts are, for example, mineral acids, such as hydrogen chloride; suitable solvents are, for example, dichloromethane or n-decane. The reaction is, for example, carried out in the range from 0 to 200°C, preferably from 90 to 180 °C.
Optically active amines of the formula (V) required for the above reaction and for preparation variant b) are known or can be prepared by processes known per se (cf. Tetrahedron Lett. 29 (1988) 223-224, Tetrahedron Lett. 36 (1995) 3917-3920; Synlett (1995) 142-146; Tetrahedron, Asymmetry 5 (1994) 817-820; EP-A-320898, EP-A-443606, DE-A-3426919, DE-A-4000610).

The compound of the formula (IV), or a direct precursor thereof, can be prepared, for example, as follows:
1. Reaction of a compound of the formula (II) with an amidinothiourea derivative of the formula (VI),

   H₂N-C(=NH)-NH-C(=NH)-S-Z¹ (VI)

   in which Z¹ is (C₁-C₄)alkyl or phenyl-(C₁-C₄)alkyl gives compounds of the formula (IV) in which Z = -SZ¹.
2. Reaction of an amidine of the formula (VII) or an acid addition salt thereof, 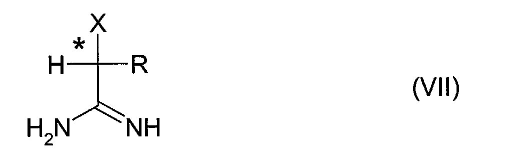 in which R and X are as defined in formula (I),
   with an N-cyanodithioiminocarbonate of the formula (VIII),

   NC-N=C(S-Z²)₂ (VIII)

   in which Z² is (C₁-C₄)alkyl or phenyl-(C₁-C₄)alkyl gives compounds of the formula (IV) in which Z = -S-Z².
3. Reaction of an alkali metal dicyanamide with a carboxylic acid derivative of the formula (II) mentioned gives compounds of the formula (IV) in which Z = NH₂.
4. Reaction of trichloroacetonitrile with a nitrile of the formula (IX), 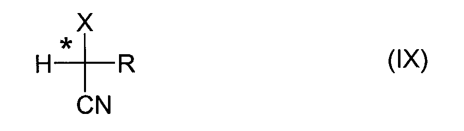 in which R and X are as defined in formula (I), initially gives compounds of the formula (X), 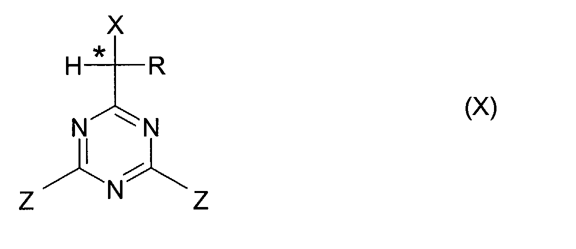
in which Z and Z³ are each CCl₃ which, by subsequent reaction with ammonia, gives compounds of the formula (IV) in which Z = CCl₃.

The reaction of the carboxylic acid derivatives of the formula (II) with the amidinothiourea derivatives of the formula (VI) is preferably carried out base-catalysed in an organic solvent, such as, for example, acetone, THF, dioxane, acetonitrile, DMF, methanol, ethanol, at temperatures from -10 °C to the boiling point of the solvent, preferably at from 0 °C to 20 °C. However, the reaction can also be carried out in water or in aqueous solvent mixtures with one or more of the abovementioned organic solvents. If the compound of formula (VI) is employed as acid addition salt, it can, if appropriate, be liberated in situ using a base. Suitable bases or basic catalysts are alkali metal hydroxides, alkali metal hydrides, alkali metal carbonates, alkali metal alkoxides, alkaline earth metal hydroxides, alkaline earth metal hydrides, alkaline earth metal carbonates or organic bases, such as triethylamine or 1,8-diazabicyclo[5.4.0]undec-7-ene (DBU). The base in question is, for example, employed in the range from 1 to 3 molar equivalents, based on the compound of the formula (VI). Compounds of the formula (II) and (VI) can be employed, for example, in equimolar amounts, or with an excess of up to 2 molar equivalents of the compound of the formula (II). In principle, the corresponding processes are known from the literature (compare: H. Eilingsfeld, H. Scheuermann, Chem. Ber.; 1967, 100, 1874); the corresponding intermediates of the formula (IV) are novel.

The reaction of the amidines of the formula (VII) with N-cyanodithioiminocarbonates of the formula (VIII) is preferably carried out base-catalysed in an inert organic solvent, such as, for example, acetonitrile, DMF, dimethylacetamide (DMA), N-methylpyrrolidone (NMP), methanol and ethanol, at temperatures from -10 °C to the boiling point of the solvent, preferably at from 20 °C to 80 °C. If (VII) is employed as acid addition salt, it can, if appropriate, be liberated in situ using a base. Suitable bases or basic catalysts are alkali metal hydroxides, alkali metal hydrides, alkali metal carbonates, alkali metal alkoxides, alkaline earth metal hydroxides, alkaline earth metal hydrides, alkaline earth metal carbonates or organic bases, such as triethylamine or 1,8-diazabicyclo[5.4.0]undec-7-ene (DBU). The base in question is employed, for example, in the range from 1 to 3 molar equivalents, based on the compound of the formula (VIII), and compounds of the formulae (VII) and (VIII) can generally be employed in equimolar amounts or with an excess of 2 molar equivalents of the compound of the formula (II). In principle, the corresponding processes are known from the literature (compare: T.A. Riley, W.J. Henney, N.K. Dalley, B.E. Wilson, R.K. Robins; J. Heterocyclic Chem.; 1986, 23 (6), 1706-1714); the corresponding intermediates of the formula (IV) are novel.

Intermediates of the formula (X) where Z = chlorine can be prepared by reacting alkali metal dicyanamide with a carboxylic acid derivative of the formula (II), Fu then preferably being the functional group carbonyl chloride or carboxamide. The reaction of the reaction components is carried out, for example, acid-catalysed in an inert organic solvent, such as, for example, toluene, chlorobenzene, chlorinated hydrocarbons, at temperatures between -10 °C and the boiling point of the solvent, preferably at from 20 °C to 80 °C, where the intermediates formed can be chlorinated in situ using a suitable chlorinating agent, such as, for example, phosphorus oxychloride. Suitable acids are, for example, hydrohalic acids, such as HCl, or else Lewis acids, such as, for example, AlCl₃ or BF₃ (compare US-A-5095113).

Intermediates of the formula (X) where Z, Z³ = trihalomethyl can be prepared by reacting the corresponding trihaloacetonitriles with a carbonitrile of the formula (IX). The reaction of the reaction components is carried out, for example, acid-catalysed in an inert organic solvent, such as, for example, toluene, chlorobenzene, chlorinated hydrocarbons, at temperatures between -40 °C and the boiling point of the solvent, preferably at from -10 °C to 30 °C. Suitable acids are, for example, hydrohalic acids, such as HCl, or else Lewis acids, such as, for example, AlCl₃ or BF₃ (cf. EP-A-130939).

Intermediates of the formula (IV), in which Z = (C₁-C₄)alkylmercapto or unsubstituted phenyl-(C₁-C₄)alkylmercapto, can be converted in an inert organic solvent, such as, for example toluene, chlorobenzene, chlorinated hydrocarbons or others, at temperatures between -40 °C and the boiling point of the solvent, preferably at from 20 °C to 80 °C, with a suitable chlorinating agent, such as, for example elemental chlorine or phosphorus oxychloride, into more reactive chlorotriazines of the formula (IV), in which Z = Cl (cf. J.K. Chakrabarti, D.E. Tupper; Tetrahedron 1975, 31(16), 1879-1882).

Intermediates of the formula (IV), in which Z = (C₁-C₄)alkylmercapto or unsubstituted or substituted phenyl-(C₁-C₄)alkylmercapto or (C₁-C₄)alkylphenylthio, can be oxidised in a suitable solvent, such as, for example, chlorinated hydrocarbons, acetic acid, water, alcohols, acetone or mixtures thereof at temperatures between 0 °C and the boiling point of the solvent, preferably at from 20 °C to 80 °C, with a suitable oxidising agent, such as, for example, m-chloroperbenzoic acid, hydrogen peroxide, potassium peroxomonosulfate (compare: T.A. Riley, W.J. Henney, N.K. Dalley, B.E. Wilson, R.K. Robins; J. Heterocyclic Chem.; 1986, 23 (6), 1706-1714).

For preparation variant c), it is possible to use customary methods for optical resolutions (cf. Textbooks of Stereochemistry), for example following processes for separating mixtures into diastereomers, for example physical processes, such as crystallization, chromatographic processes, in particular column chromatography and high pressure liquid chromatography, distillation, if appropriate under reduced pressure, extraction and other processes, it is possible to separate the remaining mixtures of enantiomers, generally by chromatographic separation on chiral solid phases. Suitable for preparative amounts or use on an industrial scale are processes such as the crystallization of diastereomeric salts which can be obtained from the compounds (I) using optically active acids and, if appropriate, provided that acidic groups are present, using optically active bases.

Optically active acids which are suitable for optical resolution by crystallisation of diastereomeric salts are, for example, camphorsulfonic acid, camphoric acid, bromocamphorsulfonic acid, quinic acid, tartaric acid, dibenzoyltartaric acid and other analogous acids; suitable optically active bases are, for example, quinine, cinchonine, quinidine, brucine, 1-phenylethylamine and other analogous bases.

The crystallisations are then in most cases carried out in aqueous or aqueous-organic solvents, where the diastereomer which is less soluble precipitates first, if appropriate after seeding. One enantiomer of the compound of the formula (I) is then liberated from the precipitated salt, or the other is liberated from the crystals, by acidification or using base

The following acids, for example, are suitable for preparing the acid addition salts of the compounds of the formula (I): hydrohalic acids, such as hydrochloric acid or hydrobromic acid, furthermore phosphoric acid, nitric acid, sulfuric acid, mono- or bifunctional carboxylic acids and hydroxycarboxylic acids, such as acetic acid, maleic acid, succinic acid, fumaric acid, tartaric acid, citric acid, salicylic acid, sorbic acid or lactic acid, and also sulfonic acids, such as p-toluenesulfonic acid and 1,5-naphthalenedisulfonic acid. The acid addition compounds of the formula (I) can be obtained in a simple manner by the customary methods for forming salts, for example by dissolving a compound of the formula (I) in a suitable organic solvent, such as, for example, methanol, acetone, methylene chloride or benzene, and adding the acid at temperatures from 0 to 100 °C, and they can be isolated in a known manner, for example by filtration, and, if appropriate, purified by washing with an inert organic solvent.

The base addition salts of the compounds of the formula (I) are preferably prepared in inert polar solvents, such as, for example, water, methanol or acetone, at temperatures from 0 to 100 °C. Examples of bases which are suitable for the preparation of the salts according to the invention are alkali metal carbonates, such as potassium carbonate, alkali metal hydroxides and alkaline earth metal hydroxides, for example NaOH or KOH, alkali metal hydrides and alkaline earth metal hydrides, for example NaH, alkali metal alkoxides and alkaline earth metal alkoxides, for example sodium methoxide or potassium tert-butoxide, or ammonia or ethanolamine. Quaternary ammonium salts can be obtained by cation exchange or condensation with quaternary ammonium salts of the formula [NRR'R"R"']⁺X-, in which R, R', R" and R''' independently of one another are (C₁-C₄)alkyl, phenyl or benzyl and X- is an anion, for example Cl⁻ or OH⁻.

Solvents referred to as "inert solvents" in the above process variants are to be understood as meaning in each case solvents which are inert under the reaction conditions in question, but which need not be inert under all reaction conditions.

A collection of compounds of the formula (I) which can be synthesised by the abovementioned processes may also be prepared in a parallel manner where the process may be carried out manually, partially automated or fully automated. In this case, it is possible to automate the procedure of the reaction, the work-up or the purification of the products or of the intermediates. In total, this is to be understood as meaning a procedure as is described, for example, by S.H. DeWitt in "Annual Reports in Combinatorial Chemistry and Molecular Diversity: Automated Synthesis", Volume 1, Verlag Escom, 1997, pages 69 to 77.

A number of commercially available apparatuses as are offered by, for example, Stem Corporation, Woodrolfe Road, Tollesbury, Essex, CM9 8SE, England and H+P Labortechnik GmbH, Bruckmannring 28, 85764 Oberschleißheim, Germany may be used for the parallel procedure of the reaction and work-up. For the parallel purification of compounds (I), or of intermediates obtained during the preparation, use may be made, inter alia, of chromatography apparatuses, for example those from ISCO, Inc., 4700 Superior Street, Lincoln, NE 68504, USA. The apparatuses mentioned lead to a modular procedure in which the individual process steps are automated, but manual operations have to be performed between the process steps. This can be avoided by employing semi-integrated or fully integrated automation systems where the automation modules in question are operated by, for example, robots. Such automation systems can be obtained, for example, from Zymark Corporation, Zymark Center, Hopkinton, MA 01748, USA.

In addition to the methods described here, compounds (I) may be prepared in part or fully by solid-phase-supported methods. For this purpose, individual intermediate steps or all intermediate steps of the synthesis or of a synthesis adapted to suit the procedure in question are bound to a synthetic resin. Solid-phase-supported synthesis methods are described extensively in the specialist literature, for example Barry A. Bunin in "The Combinatorial Index", Academic Press, 1998.
The use of solid-phase-supported synthesis methods permits a series of protocols which are known from the literature and which, in turn, can be performed manually or in an automated manner. For example, the "tea-bag method" (Houghten, US 4,631,211; Houghten et al., Proc. Natl. Acad. Sci, 1985, 82, 5131-5135), in which products from IRORI, 11149 North Torrey Pines Road, La Jolla, CA 92037, USA, are employed, may be partially automated. The automation of solid-phase-supported parallel synthesis is performed successfully, for example, by apparatuses from Argonaut Technologies, Inc., 887 Industrial Road, San Carlos, CA 94070, USA or MultiSynTech GmbH, Wullener Feld 4, 58454 Witten, Germany.
The preparation methods described here give compounds (I) in the form of collections or libraries of substances. The present invention also relates to libraries of the compounds (I) which contain at least two compounds (I) and their precursors.

The compounds of the formula (I) according to the invention and their salts, hereinbelow together referred to as compounds of the formula (I) (according to the invention) or compounds (I), have excellent herbicidal activity against a broad spectrum of economically important monocotyledonous and dicotyledonous harmful plants and can be used to control the growth of these plants. The active compounds also act efficiently on perennial weeds which produce shoots from rhizomes, root stocks or other perennial organs and which are difficult to control. In this context, it is generally immaterial whether the substances are applied pre-sowing, pre-emergence or post-emergence.

Specifically, examples may be mentioned of some representatives of the monocotyledonous and dicotyledonous weed flora which can be controlled by the compounds according to the invention, without these being a restriction to certain species.

Examples of weed species on which the active compounds act efficiently are, from amongst the monocotyledons, Agrostis, Alopecurus, Apera, Avena, Brachicaria, Bromus, Dactyloctenium, Digitaria, Echinochloa, Eleocharis, Eleusine, Festuca, Fimbristylis, Ischaemum, Lilium, Monochoria, Panicum, Paspalum, Phalaris, Pheleum, Poa, Sagittaria, Scirpus, Setaria, Sphenoclea and also Cyperus species mainly from the annual sector and, from amongst the perennial species, Agropyron, Cynodon, Imperata and Sorghum, and also perennial Cyperus species.
In the case of the dicotyledonous weed species, the spectrum of action extends to species such as, for example, Galium, Viola, Veronica, Lamium, Stellaria,
Amaranthus, Sinapis, Ipomoea, Matricaria, Abutilon and Sida from amongst the annuals, and Convolvulus, Cirsium, Rumex and Artemisia in the case of the perennial weeds.

Moreover, a herbicidal effect is observed in connection with dicotyledonous weeds such as Ambrosia, Anthemis, Carduus, Centaurea, Chenopodium, Cirsium, Convolvulus, Datura, Emex, Galeopsis, Galinsoga, Lepidium, Lindernia, Papaver, Portlaca, Polygonum, Ranunculus, Rorippa, Rotala, Seneceio, Sesbania, Solanum, Sonchus, Taraxacum, Trifolium, Urtica and Xanthium.

The active compounds according to the invention also effect outstanding control of harmful plants which occur under the specific conditions of rice-growing such as, for example, Sagittaria, Alisma, Eleocharis, Scirpus and Cyperus.

If one or more of the compounds according to the invention are applied to the soil surface prior to germination, then the weed seedlings are either prevented completely from emerging, or the weeds grow until they have reached the cotyledon stage but then their growth stops, and, eventually, after three to four weeks have elapsed, they die completely.

If one or more of the active compounds are applied post-emergence to the green parts of the plants, growth also stops drastically a very short time after the treatment and the weed plants remain at the developmental stage of the point in time of application, or they die completely after a certain time, so that in this manner competition from the weeds, which is harmful to the crop plants, is eliminated at a very early point in time and in a sustained manner.

Although the compounds according to the invention have an excellent herbicidal activity against monocotyledonous and dicotyledonous weeds, crop plants of economically important crops, for example wheat, barley, rye, triticale, rice, corn, sugar beet, cotton and soya, are not damaged at all, or only to a negligible extent. For these reasons, the present compounds are highly suitable for selectively controlling undesired plant growth in plantings of agriculturally useful plants including ornamental plants and turf.

The compounds of the invention may also be used to control the growth of weeds, especially those indicated above, at loci which are not crop-growing areas but in which the control of weeds is nevertheless desirable.

Examples of such non-crop-growing areas include airfields, industrial sites, railways, roadside verges, the verges of rivers, irrigation and other waterways, scrublands and fallow, set aside or uncultivated land, in particular where it is desired to control the growth of weeds in order to reduce fire risks. When used for such purposes in which a total herbicidal effect is frequently desired, the active compounds are normally applied at dosage rates higher than those used in crop-growing areas as hereinbefore or hereinbelow described or in combination with one of the suitable active compounds hereinbelow described. The precise dosage of compounds of the invention and of combination partner compounds will depend upon the nature of the vegetation treated and the effect sought.

In addition, the substances according to the invention have outstanding growth-regulating properties in crop plants. They engage in the plant metabolism in a regulating manner and can thus be employed for the targeted control of plant constituents and for facilitating harvesting, such as for example by provoking desiccation and stunted growth. Furthermore, they are also suitable for generally regulating and inhibiting undesirable vegetative growth, without destroying the plants in the process. Inhibition of vegetative growth plays an important role in many monocotyledonous and dicotyledonous crops because lodging can be reduced hereby, or prevented completely.

Owing to their herbicidal and plant-growth-regulatory properties, the active compounds can also be employed for controlling harmful plants in crops of known or still to be developed genetically engineered plants. The transgenic plants generally have particularly advantageous properties, for example resistance to certain pesticides, in particular certain herbicides, resistance to plant diseases or causative organisms of plant diseases, such as certain insects or microorganisms such as fungi, bacteria or viruses. Other particular properties relate, for example, to the quantity, quality, storage stability, composition and to specific ingredients of the harvested product. Thus, transgenic plants having an increased starch content or a modified quality of the starch or those having a different fatty acid composition of the harvested product are known.

The use of the compounds of the formula (I) according to the invention or their salts in economically important transgenic crops of useful and ornamental plants, for example of cereals, such as wheat, barley, rye, oats, millet, rice, manioc and corn, or else in crops of sugar beet, cotton, soy, oilseed rape, potato, tomato, pea and other vegetable species is preferred.

The compounds of the formula (I) can preferably be used as herbicides in crops of useful plants which are resistant or which have been made resistant by genetic engineering toward the phytotoxic effects of the herbicides.

Conventional ways of preparing novel plants which have modified properties compared to known plants comprise, for example, traditional breeding methods and the generation of mutants. Alternatively, novel plants having modified properties can be produced with the aid of genetic engineering methods (see, for example, EP-A-0221044, EP-A-0131624). For example, there have been described several cases of
- genetically engineered changes in crop plants in order to modify the starch synthesised in the plants (for example WO 92/11376, WO 92/14827 and WO 91/19806),
- transgenic crop plants which are resistant to certain herbicides of the glufosinate-type (cf., for example, EP-A-0242236, EP-A-0242246) or glyphosate-type (WO 92/00377), or of the sulfonylurea-type (EP-A-0257993, US-A-5013659),
- transgenic crop plants, for example cotton, having the ability to produce Bacillus thuringiensis toxins (Bt toxins) which impart resistance to certain pests to the plants (EP-A-0142924, EP-A-0193259),
- transgenic crop plants having a modified fatty acid composition (WO 91/13972).

Numerous molecular biological techniques which allow the preparation of novel transgenic plants having modified properties are known in principle; see, for example, Sambrook et al., 1989, Molecular Cloning, A Laboratory Manual, 2nd ed. Cold Spring Harbour Laboratory Press, Cold Spring Harbour, NY; or Winnacker "Gene und Klone" [Genes and Clones], VCH Weinheim, 2nd edition 1996, or Christou, "Trends in Plant Science" 1 (1996) 423-431).

In order to carry out such genetic engineering manipulations, it is possible to introduce nucleic acid molecules into plasmids which allow a mutagenesis or a change in the sequence to occur by recombination of DNA sequences. Using the abovementioned standard processes it is possible, for example, to exchange bases, to remove partial sequences or to add natural or synthetic sequences. To link the DNA fragments with each other, it is possible to attach adaptors or linkers to the fragments.

Plant cells having a reduced activity of a gene product can be prepared, for example, by expressing at least one appropriate antisense-RNA, a sense-RNA to achieve a cosuppression effect, or by expressing at least one appropriately constructed ribozyme which specifically cleaves transcripts of the abovementioned gene product.
To this end, it is possible to employ both DNA molecules which comprise the entire coding sequence of a gene product including any flanking sequences that may be present, and DNA molecules which comprise only parts of the coding sequence, it being necessary for these parts to be long enough to cause an antisense effect in the cells. It is also possible to use DNA sequences which have a high degree of homology to the coding sequences of a gene product but which are not entirely identical.

When expressing nucleic acid molecules in plants, the synthesised protein can be localised in any desired compartment of the plant cell. However, to achieve localisation in a certain compartment, it is, for example, possible to link the coding region with DNA sequences which ensure localisation in a certain compartment. Such sequences are known to the person skilled in the art (see, for example, Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

The transgenic plant cells can be regenerated to whole plants using known techniques. The transgenic plants can in principle be plants of any desired plant species, i.e. both monocotyledonous and dicotyledonous plants.

In this manner, it is possible to obtain transgenic plants which have modified properties by over-expression, suppression or inhibition of homologous (= natural) genes or gene sequences or by expression of heterologous (= foreign) genes or gene sequences.

The compounds (I) according to the invention can preferably be used in transgenic crops which are resistant to herbicides selected from the group consisting of the sulfonylureas, glufosinate-ammonium or glyphosate-isopropylammonium and analogous active compounds.

When using the active compounds according to the invention in transgenic crops, in addition to the effects against harmful plants which can be observed in other crops, there are frequently effects which are specific for the application in the respective transgenic crop, for example a modified or specifically broadened spectrum of weeds which can be controlled, modified application rates which can be used for the application, preferably good combinability with the herbicides to which the transgenic crop is resistant, and an effect on the growth and the yield of the transgenic crop plants.

The invention therefore also provides for the use of the compounds (I) according to the invention as herbicides for controlling harmful plants in transgenic crop plants.

The use according to the invention for controlling harmful plants or for regulating the growth of plants also includes the case where the active compound of the formula (I) or a salt thereof is only formed after application to the plants, in the plant or in the soil, from a precursor substance ("Prodrug").

The compounds according to the invention can be applied in the customary formulations in the form of wettable powders, emulsifiable concentrates, sprayable solutions, dusts or granules. The invention therefore also provides herbicidal and plant-growth-regulating compositions comprising compounds of the formula (I).

The compounds of the formula (I) can be formulated in various ways depending on the prevailing biological and/or chemico-physical parameters. Examples of suitable formulation options are: wettable powders (WP), water-soluble powders (SP), water-soluble concentrates, emulsifiable concentrates (EC), emulsions (EW), such as oil-in-water and water-in-oil emulsions, sprayable solutions, suspension concentrates (SC), oil- or water-based dispersions, oil-miscible solutions, capsule suspensions (CS), dusts (DP), seed-dressing compositions, granules for broadcasting and soil application, granules (GR) in the form of microgranules, spray granules, coating granules and adsorption granules, water-dispersible granules (WG), water-soluble granules (SG), ULV formulations, microcapsules and waxes.

These individual formulation types are known in principle and are described, for example, in Winnacker-Küchler, "Chemische Technologie" [Chemical Technology], Volume 7, C. Hauser Verlag Munich, 4th edition 1986; Wade van Valkenburg, "Pesticide Formulations", Marcel Dekker, N.Y., 1973; K. Martens, "Spray Drying" Handbook, 3rd ed. 1979, G. Goodwin Ltd. London.
The necessary formulation auxiliaries, such as inert materials, surfactants, solvents and other additives, are likewise known and are described, for example, in: Watkins, "Handbook of Insecticide Dust Diluents and Carriers", 2nd ed., Darland Books, Caldwell N.J., H.v. Olphen, "Introduction to Clay Colloid Chemistry"; 2nd ed., J. Wiley & Sons, N.Y.; C. Marsden, "Solvents Guide"; 2nd ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte" [Surface-active ethylene oxide adducts], Wiss. Verlagsgesell., Stuttgart 1976; Winnacker-Küchler, "Chemische Technologie" [Chemical Technology], Volume 7, C. Hauser Verlag Munich, 4th edition 1986.

Based on these formulations it is also possible to produce combinations with other pesticidally active substances, for example insecticides, acaricides, nematicides, bactericides, herbicides and fungicides, and also with safeners, fertilisers and/or growth regulators, for example in the form of a ready-mix or tank mix.
Wettable powders are preparations which are uniformly dispersible in water and which, in addition to the active compound and as well as a diluent or inert substance, also contain surfactants of ionic and/or nonionic type (wetting agents, dispersants), for example polyethoxylated alkyl phenols, polyethoxylated fatty alcohols, polyethoxylated fatty amines, fatty alcohol polyglycol ether sulfates, alkanesulfonates, alkylbenzenesulfonates, sodium ligninsulfonate, sodium 2,2'-dinaphthylmethane-6,6'-disulfonate, sodium dibutylnaphthalenesulfonate or else sodium oleoylmethyltaurinate. To prepare the wettable powders, the herbicidally active compounds are finely ground, for example in customary apparatuses such as hammer mills, fan mills and air-jet mills, and are mixed simultaneously or subsequently with the formulation auxiliaries.

Emulsifiable concentrates are prepared by dissolving the active compound in an organic solvent, for example butanol, cyclohexanone, dimethylformamide, xylene or else relatively high-boiling aromatics or hydrocarbons or mixtures of the organic solvents, with the addition of one or more surfactants of ionic and/or nonionic type (emulsifiers). Examples of emulsifiers which can be used are calcium alkylarylsulfonates, such as Ca dodecylbenzenesulfonate, or nonionic emulsifiers, such as fatty acid polyglycol esters, alkylaryl polyglycol ethers, fatty alcohol polyglycol ethers, propylene oxide-ethylene oxide condensation products, alkyl polyethers, sorbitan esters, for example sorbitan fatty acid esters or polyoxyethylene sorbitan esters, for example polyoxyethylene sorbitan fatty acid esters.

Dusts are obtained by grinding the active compound with finely divided solid substances, for example talc, natural clays, such as kaolin, bentonite and pyrophyllite, or diatomaceous earth.

Suspension concentrates can be water- or oil-based. They can be prepared, for example, by wet milling using commercially customary bead mills, with or without the addition of surfactants as already mentioned above, for example, in the case of the other formulation types.

Emulsions, for example oil-in-water emulsions (EW), can be prepared for example by means of stirrers, colloid mills and/or static mixers using aqueous organic solvents and, if desired, surfactants as already mentioned above, for example, in the case of the other formulation types.

Granules can be prepared either by spraying the active compound onto adsorptive, granulated inert material or by applying active-compound concentrates to the surface of carriers such as sand, kaolinites or granulated inert material, by means of adhesive binders, for example polyvinyl alcohol, sodium polyacrylate or else mineral oils. Suitable active compounds can also be granulated in the manner which is customary for the preparation of fertiliser granules, if desired as a mixture with fertilisers.

Water-dispersible granules are generally prepared by the customary processes, such as spray-drying, fluidised-bed granulation, disk granulation, mixing using highspeed mixers, and extrusion without solid inert material. For the preparation of disk, fluidised-bed, extruder and spray granules, see for example processes in "Spray-Drying Handbook" 3rd ed. 1979, G. Goodwin Ltd., London; J.E. Browning, "Agglomeration", Chemical and Engineering 1967, pages 147 ff; "Perry's Chemical Engineer's Handbook", 5th ed., McGraw-Hill, New York 1973, pp. 8-57.

For further details on the formulation of crop protection products, see for example G.C. Klingman, "Weed Control as a Science", John Wiley and Sons., Inc., New York, 1961, pages 81-96 and J.D. Freyer, S.A. Evans, "Weed Control Handbook", 5th ed., Blackwell Scientific Publications, Oxford, 1968, pages 101-103.

The agrochemical formulations generally contain from 0.1 to 99% by weight, in particular from 0.1 to 95% by weight, of active compound of the formula (I).
In wettable powders the concentration of active compound is, for example, from about 10 to 90% by weight, the remainder to 100% by weight consisting of customary formulation constituents. In emulsifiable concentrates the concentration of active compound can be from about 1 to 90%, preferably from 5 to 80%, by weight. Formulations in the form of dusts contain from 1 to 30% by weight of active compound, preferably most commonly from 5 to 20% by weight of active compound, while sprayable solutions contain from about 0.05 to 80%, preferably from 2 to 50%, by weight of active compound. In the case of water-dispersible granules, the content of active compound depends partly on whether the active compound is in liquid or solid form and on the granulation auxiliaries, fillers, etc. that are used. In water-dispersible granules the content of active compound, for example, is between 1 and 95% by weight, preferably between 10 and 80% by weight.

In addition, said formulations of active compound may comprise the tackifiers, wetting agents, dispersants, emulsifiers, penetrants, preservatives, antifreeze agents, solvents, fillers, carriers, colorants, antifoams, evaporation inhibitors and pH and viscosity regulators which are customary in each case.

The compounds of the formula (I) or their salts can be used as such or combined in the form of their preparations (formulations) with other pesticidally active compounds, such as, for example, insecticides, acaricides, nematicides, herbicides, fungicides, safeners, fertilisers and/or growth regulators, for example as finished formulations or tank mixes.

Suitable active compounds which can be combined with the active compounds according to the invention in mixed formulations or in a tank mix are, for example, known active compounds, as described, for example, in Weed Research 26, 441-445 (1986), or in "The Pesticide Manual", 12th edition, The British Crop Protection Council and the Royal Soc. of Chemistry, 2000, and in the literature cited therein, or in the "Compendium of Pesticide Common Names" (available from the Internet). For example, the following active compounds may be mentioned as herbicides or plant-growth regulators which are known and which can be combined with the compounds of the formula (I); the compounds are either referred to by the "common name" in accordance with the International Organisation for Standardisation (ISO) or by the chemical names, if appropriate together with a customary code number:
acetochlor; acifluorfen(-sodium); aclonifen; AKH 7088, i.e. [[[1-[5-[2-chloro-4-(trifluoromethyl)phenoxy]-2-nitrophenyl]-2-methoxyethylidene]amino]oxy]acetic acid and its methyl ester; alachlor; alloxydim(-sodium); ametryn; amicarbazone, amidochlor, amidosulfuron; amitrol; AMS, i.e. ammonium sulfamate; anilofos; asulam; atrazine; azafenidin; azimsulfuron (DPX-A8947); aziprotryn; barban; BAS 516 H, i.e. 5-fluoro-2-phenyl-4H-3,1-benzoxazin-4-one; beflubutamid; benazolin(-ethyl); benfluralin; benfuresate; bensulfuron(-methyl); bensulide; bentazone(-sodium); benzobicyclone; benzofenap; benzofluor; benzoylprop(-ethyl); benzthiazuron; bialaphos (bilanafos); bifenox; bispyribac(-sodium); bromacil; bromobutide; bromofenoxim; bromoxynil; bromuron; buminafos; busoxinone; butachlor; butafenacil; butamifos; butenachlor; buthidazole; butralin; butroxydim; butylate; cafenstrole (CH-900); carbetamide; carfentrazone(-ethyl); caloxydim, CDAA, i.e. 2-chloro-N,N-di-2-propenylacetamide; CDEC, i.e. 2-chloroallyl diethyldithiocarbamate; chlomethoxyfen; chloramben; chlorazifop-butyl; chlorbromuron; chlorbufam; chlorfenac; chlorflurenol-methyl; chloridazon; chlorimuron(-ethyl); chlornitrofen; chlorotoluron; chloroxuron; chlorpropham; chlorsulfuron; chlorthal-dimethyl; chlorthiamid; chlortoluron, cinidon(-methyl oder - ethyl), cinmethylin; cinosulfuron; clethodim; clefoxydim, clodinafop and its ester derivatives (for example clodinafop-propargyl); clomazone; clomeprop; cloproxydim; clopyralid; clopyrasulfuron(-methyl); cloransulam(-methyl); cumyluron (JC 940); cyanazine; cycloate; cyclosulfamuron (AC 104); cycloxydim; cycluron; cyhalofop and its ester derivatives (for example butyl-ester, DEH-112); cyperquat; cyprazine; cyprazole; daimuron; 2,4-D; 2,4-DB; dalapon; dazomet, desmedipham; desmetryn; di-allate; dicamba; dichlobenil; dichlorprop(-P); diclofop and its esters such as diclofop-methyl; diclosulam, diethatyl(-ethyl); difenoxuron; difenzoquat; diflufenican; diflufenzopyr; dimefuron; dimepiperate; dimethachlor; dimethametryn; dimethenamid (SAN-582H); dimethenamid(-P); dimethazone, dimethipin; dimexyflam, dimetrasulfuron, dinitramine; dinoseb; dinoterb; diphenamid; dipropetryn; diquat; dithiopyr; diuron; DNOC; eglinazine-ethyl; EL 77, i.e. 5-cyano-1-(1,1-dimethylethyl)-N-methyl-1 H-pyrazole-4-carboxamide; endothal; epoprodan, EPTC; esprocarb; ethalfluralin; ethametsulfuron-methyl; ethidimuron; ethiozin; ethofumesate; ethoxyfen and its esters (for example ethyl ester, HC-252), ethoxysulfuron, etobenzanid (HW 52); F5231, i.e. N-[2-chloro-4-fluoro-5-[4-(3-fluoropropyl)-4,5-dihydro-5-oxo-1H-tetrazol-1-yl]-phenyl]ethanesulfonamide; fenoprop; fenoxan, fenoxaprop and fenoxaprop-P and their esters, for example fenoxaprop-P-ethyl and fenoxaprop-ethyl; fenoxydim; fentrazamide; fenuron; flamprop(-methyl or -isopropyl or -isopropyl-L); flazasulfuron; florasulam; fluazifop and fluazifop-P and their esters, for example fluazifop-butyl and fluazifop-P-butyl; fluazolate, flucarbazone(-sodium); fluchloralin; flufenacet (FOE 5043), flufenpyr, flumetsulam; flumeturon; flumiclorac(-pentyl); flumioxazin (S-482); flumipropyn; fluometuron; fluorochloridone, fluorodifen; fluoroglycofen(-ethyl); flupoxam (KNW-739); flupropacil (UBIC-4243); fluproanate, flupyrsulfuron(-methyl, or -sodium); flurenol(-butyl); fluridone; flurochloridone; fluroxypyr(-meptyl); flurprimidol, flurtamone; fluthiacet(-methyl); fluthiamide (flufenacet); fomesafen; foramsulfuron; fosamine; furilazole (MON 13900), furyloxyfen; glufosinate(-ammonium); glyphosate(-isopropylammonium); halosafen; halosulfuron(-methyl) and its esters (for example the methyl ester, NC-319); haloxyfop and its esters; haloxyfop-P (= R-haloxyfop) and its esters; HC-252 (diphenylether), hexazinone; imazamethabenz(-methyl); imazamethapyr; imazamox; imazapic, imazapyr; imazaquin and salts such as the ammonium salts; imazethamethapyr; imazethapyr, imazosulfuron; indanofan; iodosulfuron-(methyl)-(sodium), ioxynil; isocarbamid; isopropalin; isoproturon; isouron; isoxaben; isoxachlortole; isoxaflutole; isoxapyrifop; karbutilate; lactofen; lenacil; linuron; MCPA; MCPB; mecoprop; mefenacet; mefluidid; mesosulfuron(-methyl); mesotrione; metam, metamifop, metamitron; metazachlor; methabenzthiazuron; methazole; methoxyphenone; methyldymron; metobenzuron, metobromuron; (S-)metolachlor; metosulam (XRD 511); metoxuron; metribuzin; metsulfuron-methyl; MK-616; molinate; monalide; monocarbamide dihydrogensulfate; monolinuron; monuron; MT 128, i.e. 6-chloro-N-(3-chloro-2-propenyl)-5-methyl-N-phenyl-3-pyridazinamine; MT 5950, i.e. N-[3-chloro-4-(1-methylethyl)-phenyl]-2-methylpentanamide; naproanilide; napropamide; naptalam; NC 310, i.e. 4-(2,4-dichlorobenzoyl)-1-methyl-5-benzyloxypyrazole; neburon; nicosulfuron; nipyraclophen; nitralin; nitrofen; nitrofluorfen; norflurazon; orbencarb; oryzalin; oxadiargyl (RP-020630); oxadiazone; oxasulfuron; oxaziclomefone; oxyfluorfen; paraquat; pebulate; pelargonic acid; pendimethalin; penoxulam. Pentanochlor, pentoxazone; perfluidone; pethoxamid, phenisopham; phenmedipham; picloram; picolinafen; piperophos; piributicarb; pirifenop-butyl; pretilachlor; primisulfuron(-methyl); procarbazone(-sodium); procyazine; prodiamine; profluazole, profluralin; proglinazine(-ethyl); prometon; prometryn; propachlor; propanil; propaquizafop; propazine; propham; propisochlor; propoxycarbazone(-sodium), propyzamide; prosulfalin; prosulfocarb; prosulfuron (CGA-152005); prynachlor; pyraclonil, pyraflufen(-ethyl); pyrazolinate; pyrazon; pyrazosulfuron(-ethyl); pyrazoxyfen; pyribenzoxim; pyributicarb; pyridafol; pyridate; pyriftalid, pyrimidobac(-methyl); pyrithiobac(-sodium) (KIH-2031 ); pyroxofop and its esters (for example propargyl ester); quinclorac; quinmerac; quinoclamine, quinofop and its ester derivatives, quizalofop and quizalofop-P and their ester derivatives, for example quizalofop-ethyl; quizalofop-P-tefuryl and -ethyl; renriduron; rimsulfuron (DPX-E 9636); S 275, i.e. 2-[4-chloro-2-fluoro-5-(2-propynyloxy)phenyl]-4,5,6,7-tetrahydro-2H-indazole; secbumeton; sethoxydim; siduron; simazine; simetryn; SN 106279, i.e. 2-[[7-[2-chloro-4-(trifluoromethyl)phenoxy]-2-naphthalenyl]oxy]propanoic acid and its methyl ester; sulcotrione; sulfentrazone (FMC-97285, F-6285); sulfazuron; sulfometuron(-methyl); sulfosate (ICI-A0224); sulfosulfuron; TCA; tebutam (GCP-5544); tebuthiuron; tepraloxydim; terbacil; terbucarb; terbuchlor; terbumeton; terbuthylazine; terbutryn; TFH 450, i.e. N,N-diethyl-3-[(2-ethyl-6-methylphenyl)sulfonyl]-1H-1,2,4-triazole-1-carboxamide; thenylchlor (NSK-850); thiafluamide; thiazafluron; thiazopyr (Mon-13200); thidiazimin (SN-24085); thifensulfuron(-methyl); thiobencarb; tiocarbazil; tralkoxydim; tri-allate; triasulfuron; triaziflam; triazofenamide; tribenuron(-methyl); 2,3,6-trichlorobenzoic acid (2,3,6-TBA), triclopyr; tridiphane; trietazine; trifloxysulfuron(-sodium), trifluralin; triflusulfuron and esters (e.g. methyl ester, DPX-66037); trimeturon; tritosulfuron; tsitodef; vernolate; WL 110547, i.e. 5-phenoxy-1-[3-(trifluoromethyl)phenyl]-1H-tetrazole; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127; KIH-2023 and KIH5996.

Of particular interest is the selective control of harmful plants in crops of useful and ornamental plants. Although the compounds (I) according to the invention have very good to satisfactory selectivity in a large number of crops, it is possible in principle that phytotoxicity in the crop plants can occur in some crops and, in particular, when the compounds (I) are mixed with other herbicides which are less selective. In this respect, combinations of the compounds (I) according to the invention which contain the compounds (I), or their combinations with other herbicides or pesticides, and safeners are of particular interest. The safeners, which are employed in such amounts that they act as antidotes, reduce the phytotoxic side effects of the herbicides/pesticides used, for example in economically important crops such as cereals (wheat, barley, rye, corn, rice, millet), sugar beet, sugar cane, oilseed rape, cotton and soy, preferably cereal. Suitable safeners for the compounds (I) and their combinations with other pesticides are, for example, the following groups of compounds:
a) Compounds of the type of dichlorophenylpyrazoline-3-carboxylic acid, preferably compounds such as ethyl 1-(2,4-dichlorophenyl)-5-(ethoxycarbonyl)-5-methyl-2-pyrazoline-3-carboxylate (S1-1) ("mefenpyrdiethyl", PM, pp. 781-782), and related compounds, as described in WO 91/07874.
b) Derivatives of dichlorophenylpyrazole carboxylic acid, preferably compounds such as ethyl 1-(2,4-dichlorophenyl)-5-methylpyrazole-3-carboxylate (S1-2), ethyl 1-(2,4-dichlorophenyl)-5-isopropylpyrazole-3-carboxylate (S1-3), ethyl 1-(2,4-dichlorophenyl)-5-(1,1-dimethylethyl)pyrazole-3-carboxylate (S1-4), ethyl 1-(2,4-dichlorophenyl)-5-phenylpyrazole-3-carboxylate (S1-5) and related compounds as described in EP-A-333 131 and EP-A-269 806.
c) Compounds of the type of the triazolecarboxylic acids, preferably compounds such as fenchlorazole(ethyl ester), i.e. ethyl 1-(2,4-dichlorophenyl)-5-trichloromethyl-(1H)-1,2,4-triazole-3-carboxylate (S1-6) and related compounds as described in EP-A-174 562 and EP-A-346 620.
d) Compounds of the type of the 5-benzyl- or 5-phenyl-2-isoxazoline-3-carboxylic acid, or the 5,5-diphenyl-2-isoxazoline-3-carboxylic acid, preferably compounds such as ethyl 5-(2,4-dichlorobenzyl)-2-isoxazoline-3-carboxylate (S1-7) or ethyl 5-phenyl-2-isoxazoline-3-carboxylate (S1-8) and related compounds, as described in WO 91/08202, or ethyl 5,5-diphenyl-2-isoxazolinecarboxylate (S1-9) ("isoxadifen-ethyl") or its n-propyl ester (S1-10) or ethyl 5-(4-fluorophenyl)-5-phenyl-2-isoxazoline-3-carboxylate (S1-11), as described in the German patent application (WO-A-95/07897).
e) Compounds of the type of the 8-quinolineoxyacetic acid (S2), preferably
   1-methylhex-1-yl (5-chloro-8-quinolineoxy) acetate (common name "cloquintocet-mexyl" (S2-1) (see PM, pp. 263-264)
   1,3-dimethylbut-1-yl (5-chloro-8-quinolineoxy)acetate (S2-2),
   4-allyloxybutyl (5-chloro-8-quinolineoxy)acetate (S2-3),
   1-allyloxyprop-2-yl (5-chloro-8-quinolineoxy)acetate (S2-4),
   ethyl (5-chloro-8-quinolineoxy)acetate (S2-5),
   methyl (5-chloro-8-quinolineoxy)acetate (S2-6),
   allyl (5-chloro-8-quinolineoxy)acetate (S2-7),
   2-(2-propylideneiminoxy)-1-ethyl (5-chloro-8-quinolineoxy)acetate (S2-8),
   2-oxoprop-1-yl (5-chloro-8-quinolineoxy)acetate (S2-9)
   and related compounds, as described in EP-A-86 750, EP-A-94 349 and EP-A-191 736 or EP-A-0 492 366.
f) Compounds of the type of the (5-chloro-8-quinolineoxy)malonic acid, preferably compounds such as diethyl (5-chloro-8-quinolineoxy)malonate, diallyl (5-chloro-8-quinolineoxy)malonate, methyl ethyl
   (5-chloro-8-quinolineoxy)malonate and related compounds, as described in EP-A-0 582 198.
g) Active compounds of the type of the phenoxyacetic or -propionic acid derivatives or the aromatic carboxylic acids, such as, for example,
   2,4-dichlorophenoxyacetic acid (esters) (2,4-D),
   4-chloro-2-methylphenoxypropionic esters (mecoprop), MCPA or 3,6-dichloro-2-methoxybenzoic acid (esters) (dicamba).
h) Active compounds of the type of the pyrimidines, which are used as soil-acting safeners in rice, such as, for example,
   "fenclorim" (PM, pp. 511-512) (= 4,6-dichloro-2-phenylpyrimidine), which is known as safener for pretilachlor in sown rice.
i) Active compounds of the type of the dichloroacetamides, which are frequently used as pre-emergent safeners (soil-acting safeners), such as, for example, "dichlormid" (PM, pp. 363-364) (= N,N-diallyl-2,2-dichloroacetamide),
   "R-29148" (= 3-dichloroacetyl-2,2,5-trimethyl-1,3-oxazolidine from Stauffer), "benoxacor" (PM, pp. 102-103) (= 4-dichloroacetyl-3,4-dihydro-3-methyl-2H-1,4-benzoxazine).
   "PPG-1292" (= N-allyl-N-[(1,3-dioxolan-2-yl)methyl]dichloroacetamide from PPG Industries),
   "DK-24" (= N-allyl-N-[(allylaminocarbonyl)methyl]dichloroacetamide from Sagro-Chem),
   "AD-67" or "MON 4660" (= 3-dichloroacetyl-1-oxa-3-aza-spiro[4,5]decane from Nitrokemia or Monsanto),
   "diclonon" or "BAS145138" or "LAB145138" (= 3-dichloroacetyl-2,5,5-trimethyl-1,3-diazabicyclo[4.3.0]nonane from BASF) and
   "furilazol" or "MON 13900" (see PM, 637-638) (= (RS)-3-dichloroacetyl-5-(2-furyl)-2,2-dimethyloxazolidine).
j) Active compounds of the type of the dichloroacetone derivatives, such as, for example,
   "MG 191" (CAS-Reg. No. 96420-72-3) (= 2-dichloromethyl-2-methyl-1,3-dioxolane from Nitrokemia), which is known as safener for corn.
k) Active compounds of the type of the oxyimino compounds, which are known as seed dressings, such as, for example,
   "oxabetrinil" (PM, pp. 902-903) (= (Z)-1,3-dioxolan-2-ylmethoxyimino(phenyl)acetonitrile), which is known as seed dressing safener for millet against metolachlor damage,
   "fluxofenim" (PM, pp. 613-614) (= 1-(4-chlorophenyl)-2,2,2-trifluoro-1-ethanone O-(1,3-dioxolan-2-ylmethyl) oxime), which is known as seed dressing safener for millet against metolachlor damage,
   "cyometrinil" or "-CGA-43089" (PM, p. 1304) (= (Z)-cyanomethoxyimino(phenyl)acetonitrile), which is known as seed dressing safener for millet against metolachlor damage,
l) Active compounds of the type of the thiazolecarboxylic esters, which are known as seed dressings, such as, for example,
   "flurazol" (PM, pp. 590-591) (= benzyl 2-chloro-4-trifluoromethyl-1,3-thiazole-5-carboxylate), which is known as seed dressing safener for millet against alachlor and metolachlor damage.
m) Active compounds of the type of the naphthalenedicarboxylic acid derivatives, which are known as seed dressings, such as, for example,
   "naphthalic anhydride" (PM, p. 1342) (= 1,8-naphthalenedicarboxylic anhydride), which is known as seed dressing safener for corn against thiocarbamate herbicide damage.
n) Active compounds of the type of the chromanacetic acid derivatives, such as, for example,
   "CL 304415" (CAS-Reg. No. 31541-57-8) (= 2-(4-carboxychroman-4-yl)acetic acid from American Cyanamid), which is known as safener for corn against imidazolinone damage.
o) Active compounds which, in addition to a herbicidal action against harmful plants, also have safener action in crop plants such as rice, such as, for example,
   "dimepiperate" or "MY-93" (PM, pp. 404-405) (= S-1-methyl-1-phenylethyl piperidine-1-thiocarboxylate), which is known as safener for rice against damage by the herbicide molinate,
   "daimuron" or "SK 23" (PM, p. 330) (= 1-(1-methyl-1-phenylethyl)-3-p-tolylurea), which is known as safener for rice against damage by the herbicide imazosulfuron,
   "cumyluron" = "JC-940" (= 3-(2-chlorophenylmethyl)-1-(1-methyl-1-phenylethyl)urea, see JP-A-60087254), which is known as safener for rice against damage by some herbicides,
   "methoxyphenon" or "NK 049" (= 3,3'-dimethyl-4-methoxybenzophenone), which is known as safener for rice against damage by some herbicides, "CSB" (= 1-bromo-4-(chloromethylsulfonyl)benzene) (CAS-Reg. No. 54091-06-4 from Kumiai), which is known as safener against damage by some herbicides in rice.
p) N-Acylsulfonamides of the formula (S3) and salts thereof, 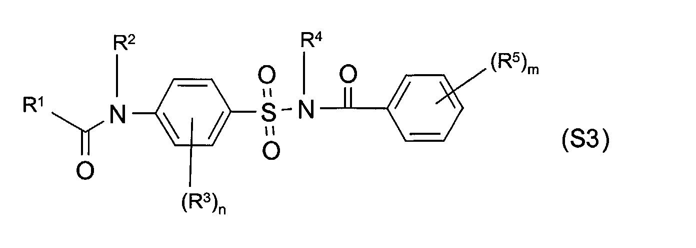 as described in WO-A-97/45016.
q) Acylsulfamoylbenzoamides of the formula (S4), if appropriate also in salt form, 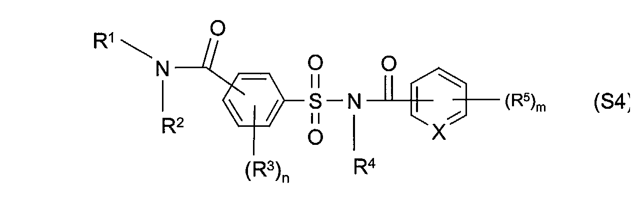 as described in the International Application No. PCT/EP98/06097.
r) Compounds of the formula (S5), 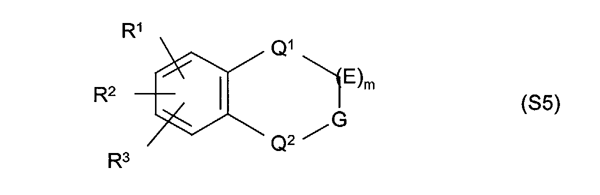 as described in WO-A 98/13 361.

The safeners include in each case the stereoisomers and the salts used in agriculture.Among the safeners mentioned, (S1-1) and (S1-9) and (S2-1), in particular (S1-1) and (S1-9), are of particular interest. Some of the safeners are already known as herbicides and consequently show, in addition to the herbicidal action against harmful plants, also protective action in connection with crop plants.

The ratios by weight of herbicide (mixture) to safener generally depend on the application rate of the herbicide and the efficacy of the safener in question and can vary within wide limits, for example in the range from 200:1 to 1:200, preferably 100:1 to 1:100, in particular 20:1 to 1:20 and most preferably 1:10 to 10:1. Analogously to the compounds (I) or their mixtures, the safeners can be formulated with other herbicides/pesticides and be provided and used as ready-mix or tank mix with the herbicides.

For use, the herbicide or herbicide safener formulations which are present in commercially available form are, if appropriate, diluted in the customary manner, for example using water in the case of wettable powders, emulsifiable concentrates, dispersions and water-dispersible granules. Preparations in the form of dusts, granules for soil application or broadcasting and sprayable solutions are usually not further diluted with other inert substances prior to use.

The application rate of the compounds of the formula (I) required varies with the external conditions, such as temperature, humidity, the nature of the herbicide used and the like. It can vary within wide limits, for example between 0.001 and 10.0 kg/ha or more of active substance, but it is preferably between 0.005 and 5 kg/ha.

For use, the formulations which are present in commercially available form are, if appropriate, diluted in the customary manner, for example using water in the case of wettable powders, emulsifiable concentrates, dispersions and water-dispersible granules. Preparations in the form of dusts, granules for soil application or broadcasting and sprayable solutions are usually not further diluted with other inert substances prior to use.

The application rate of the compounds of the formula (I) varies with the external conditions, such as temperature, humidity, the nature of the herbicides used and the like. It can vary within wide limits, for example between 0.001 and 10.0 kg/ha or more of active substance, but it is preferably between 0.005 and 5 kg/ha.

In the context of the description and the examples the amounts (including percentages) are based on weight, unless specifically defined otherwise. The terms "R" and "S" used in the context of the description and the examples for the absolute configuration at the chiral center in question of the stereoisomers of the formula (I) follows the RS nomenclature according to the Cahn-Ingold- Prelog rules.

### A. Chemical examples

In the examples which follow, quantities and percentages are weight based unless stated otherwise. Ratios of solvents are volume based.

### Example A1

### N-{4-amino-6-[(1S)-1-fluoroethyl]-1,3,5-triazin-2-yl}-N-[(1S)-1-cyclobutyl-3-phenylpropyl]amine (Compound No. 1.1 in Table 1)

A mixture of (1 S)-1-cyclobutyl-3-phenylpropylamine hydrochloride (3.00 g, 13.27 mmol) and finely powdered 1-cyanoguanidine (1.12 g, 13.27 mmol) was heated in 10 ml 1,2-dichlorobenzene for 3 hours at 160°C. The cooled mixture was filtered, washed with heptane and dried to give 1-[(S)-cyclobutyl-3-phenylpropyl]biguanide hydrochloride (4.03 g, 98% of theory) as colourless crystals.
To a solution of 4.03 g (13.00 mmol) of the above obtained 1-[(S)-cyclobutyl-3-phenylpropyl]biguanide hydrochloride in 15 ml methanol was added a solution of 327 mg (13.65 mmol) sodium in 2 ml methanol. Methyl (2S)-2-fluoropropanoate (2.88 g, 27.2 mmol) was then added and the mixture stirred for 6 hours at 20°C, then 4 hours at 65°C. The solvent was evaporated and the residue dissolved in dichloromethane. The crude material was washed with water, dried over sodium sulphate and evaporated to give N-{4-amino-6-[(1S)-1-fluoroethyl]-1,3,5-triazin-2-yl}-*N*-[(1*S*)-1-cyclobutyl-3-phenylpropyl]amine (Compound No. 1.1, 4.02 g, 92% of theory).

By proceeding in a similar manner but using 4.03 g (13.00 mmol) 1-[(S)-cyclobutyl-3-phenylpropyl]biguanide hydrochloride and racemic methyl 2-fluoropropanoate (2.88 g, 27.2 mmol) there was obtained a 1:1 mixture of N-{4-amino-6-[(1S)-1-fluoroethyl]-1,3,5-triazin-2-yl}-*N*-[(1*S*)-1-cyclobutyl-3-phenylpropyl]amine and *N*-{4-amino-6-[(1*R*)-1-fluoroethyl]-1,3,5-triazin-2-yl}-*N*-[(1*S*)-1-cyclobutyl-3-phenylpropyl]amine (Compound Nos. a mixture of 1.1 and 2.1 - 4.02 g, 92% of theory) as colourless crystals, which were analysed by chiral HPLC.

### Example A2

### rac-N-[4-amino-6-(1-fluoropropyl)-1,3,5-triazin-2-yl]-N-(1-cyclobutyl-3-phenylpropyl) amine (Compound No. 5.15)

A mixture of racemic 1-cyclobutyl-3-phenylpropylamine (0.57 g, 3.0 mmol), racemic 2-amino-4-chloro-6-(1-fluoropropyl)-1,3,5-triazine (0.51 g, 3.0 mmol) and potassium carbonate (1.11 g, 8.0 mmol) in 5 ml acetonitrile was heated under reflux for 2 hours. The cooled mixture was filtered, evaporated and purified by column chromatography (eluent: ethyl acetate-hexane = 1:1) to give racemic N-[4-amino-6-(1-fluoropropyl)-1,3,5-triazin-2-yl]-*N*-(1-cyclobutyl-3-phenylpropyl) amine (Compound No. 5.15 0.92 g, 89% of theory).

### Example A3

A 1:1 mixture of *N*-{4-amino-6-[(1*S*)-1-fluoroethyl]-1,3,5-triazin-2-yl}-*N*-[(1*S*)-1-cyclobutyl-3-phenylpropyl]amine and *N*-{4-amino-6-[(1*R*)-1-fluoroethyl]-1,3,5-triazin-2-yl}-*N*-[(1*S*)-1-cyclobutyl-3-phenylpropyl]amine as prepared in example A1 above was separated by chiral HPLC using a Chiracel™ OD column (Daicel Chemical Industries Ltd - 250x4.6 mm) and n-hexane:2-propanol (90:10 v/v) as the mobile phase. The flow rate was 1 ml/min and the eluted compounds were detected at 210 nm.
Under these conditions *N*-{4-amino-6-[(1*S*)-1-fluoroethyl]-1,3,5-triazin-2-yl}-*N*-[(1S)-1-cyclobutyl-3-phenylpropyl]amine (compound 1.1) elutes at 13.84 minutes whilst *N*-{4-amino-6-[(1*R*)-1-fluoroethyl]-1,3,5-triazin-2-yl}-*N*-[(1*S*)-1-cyclobutyl-3-phenylpropyl]amine (compound 2.1) elutes at 16.24 minutes.

The following preferred compounds of formula (I) shown in Tables 1, 2, 3, 4, 5 and 6 also form part of the present invention, and are obtained by, or analogously to, the above Examples A1, A2 and A3 or the above-described general methods.

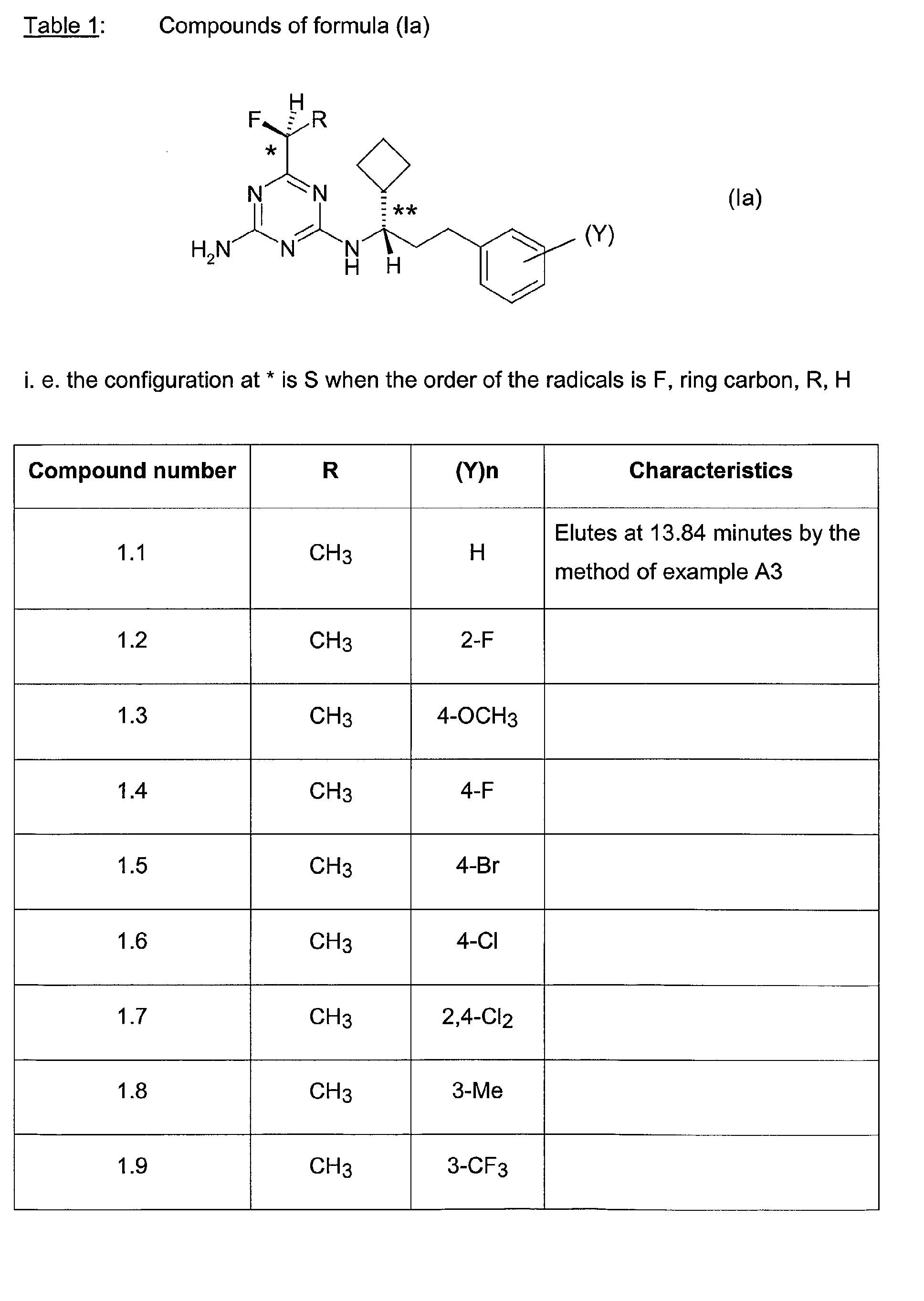

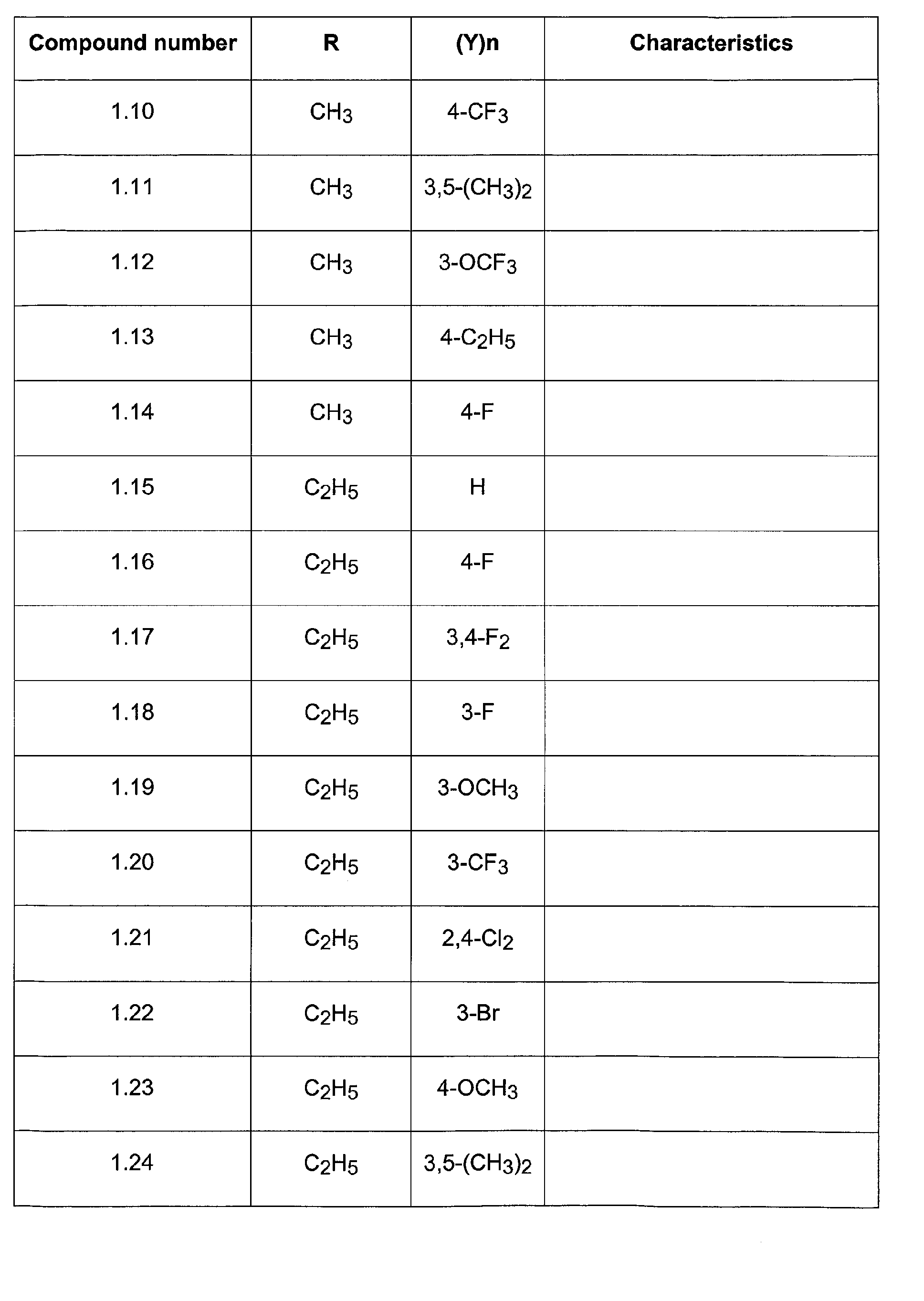

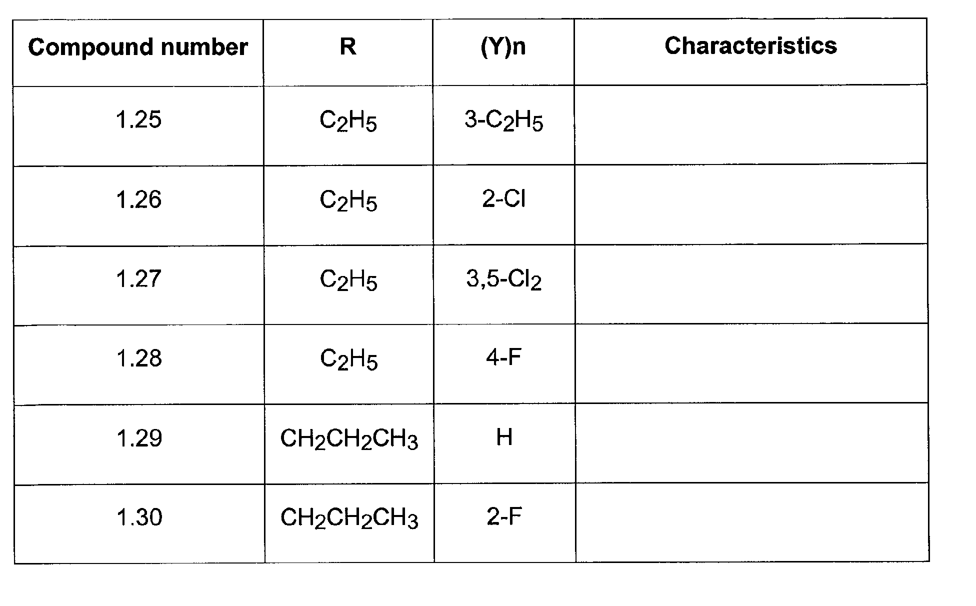

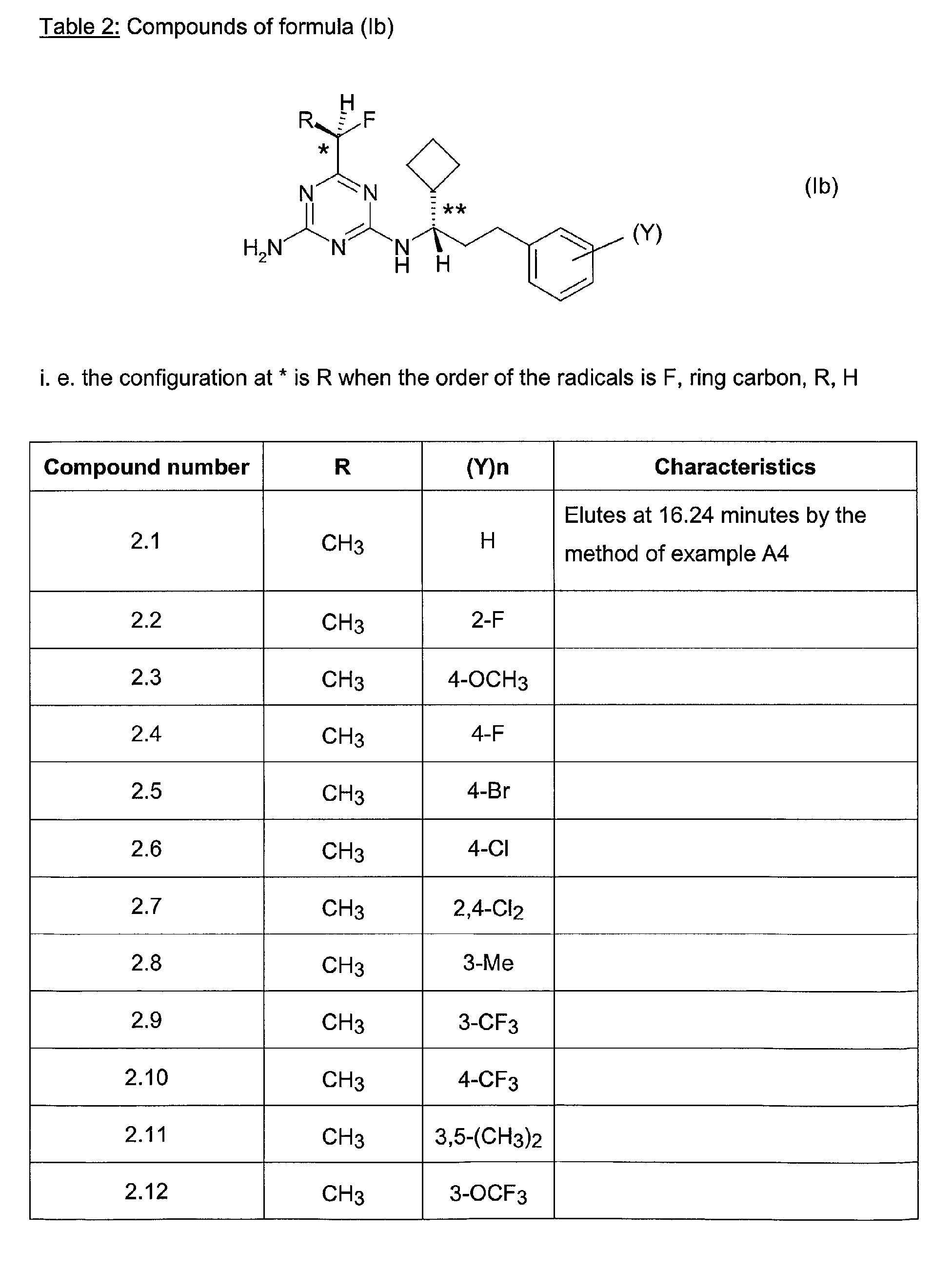

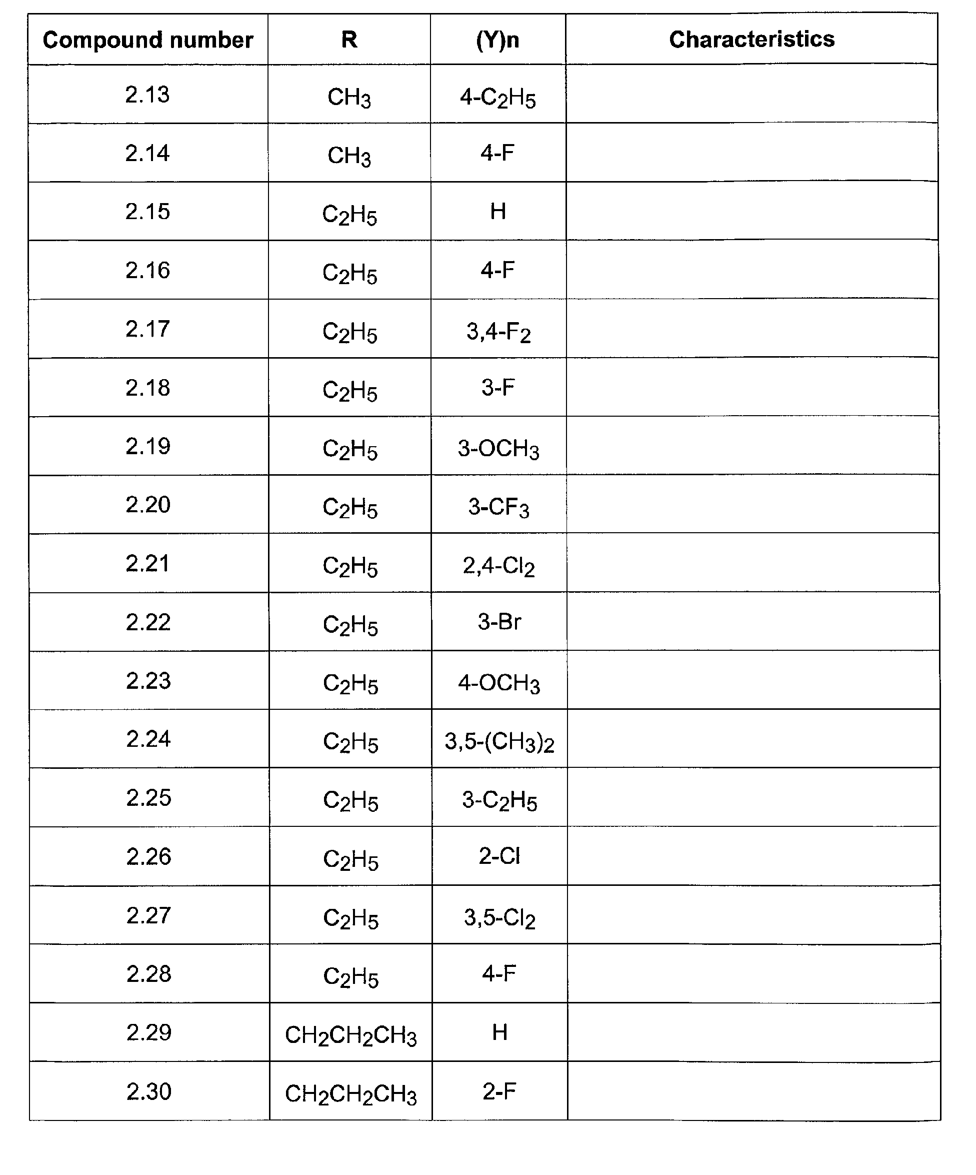

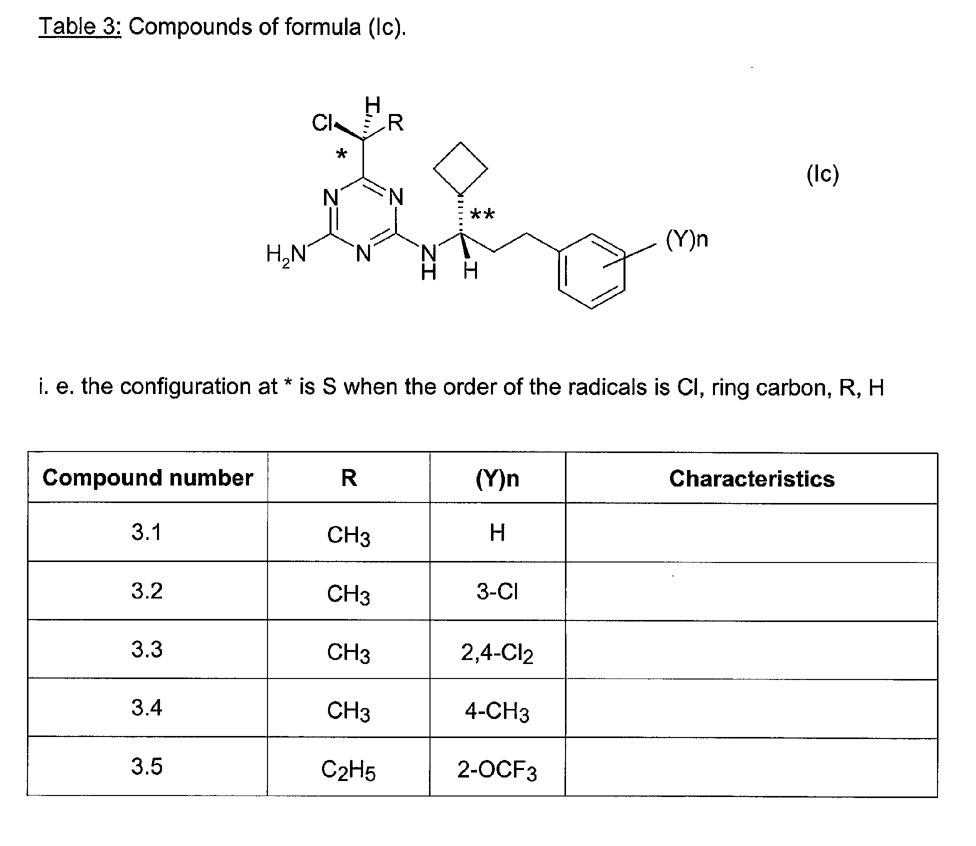

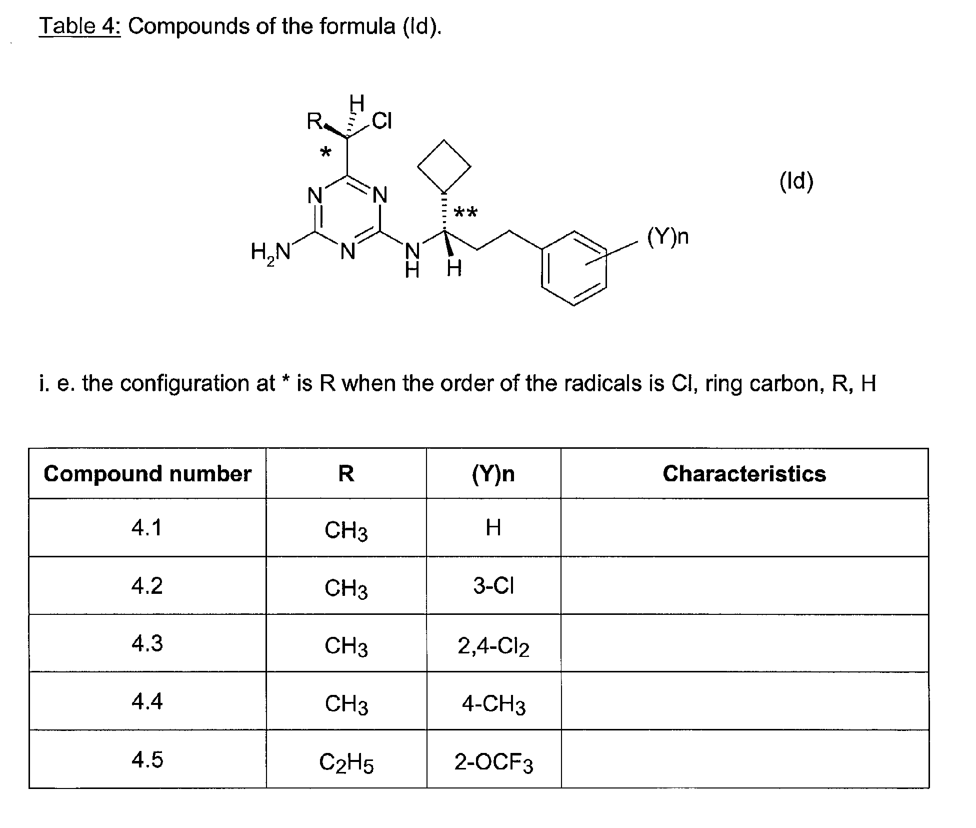

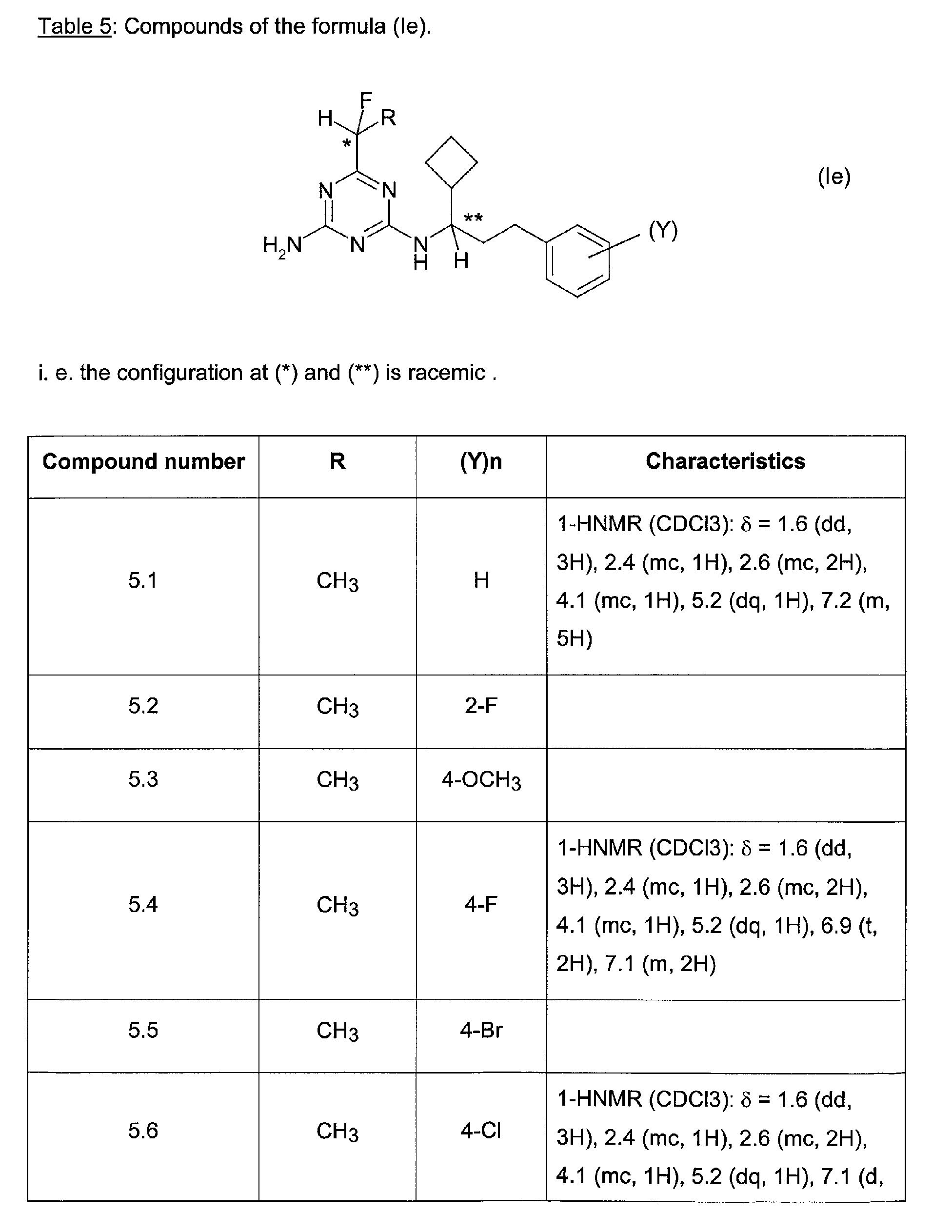

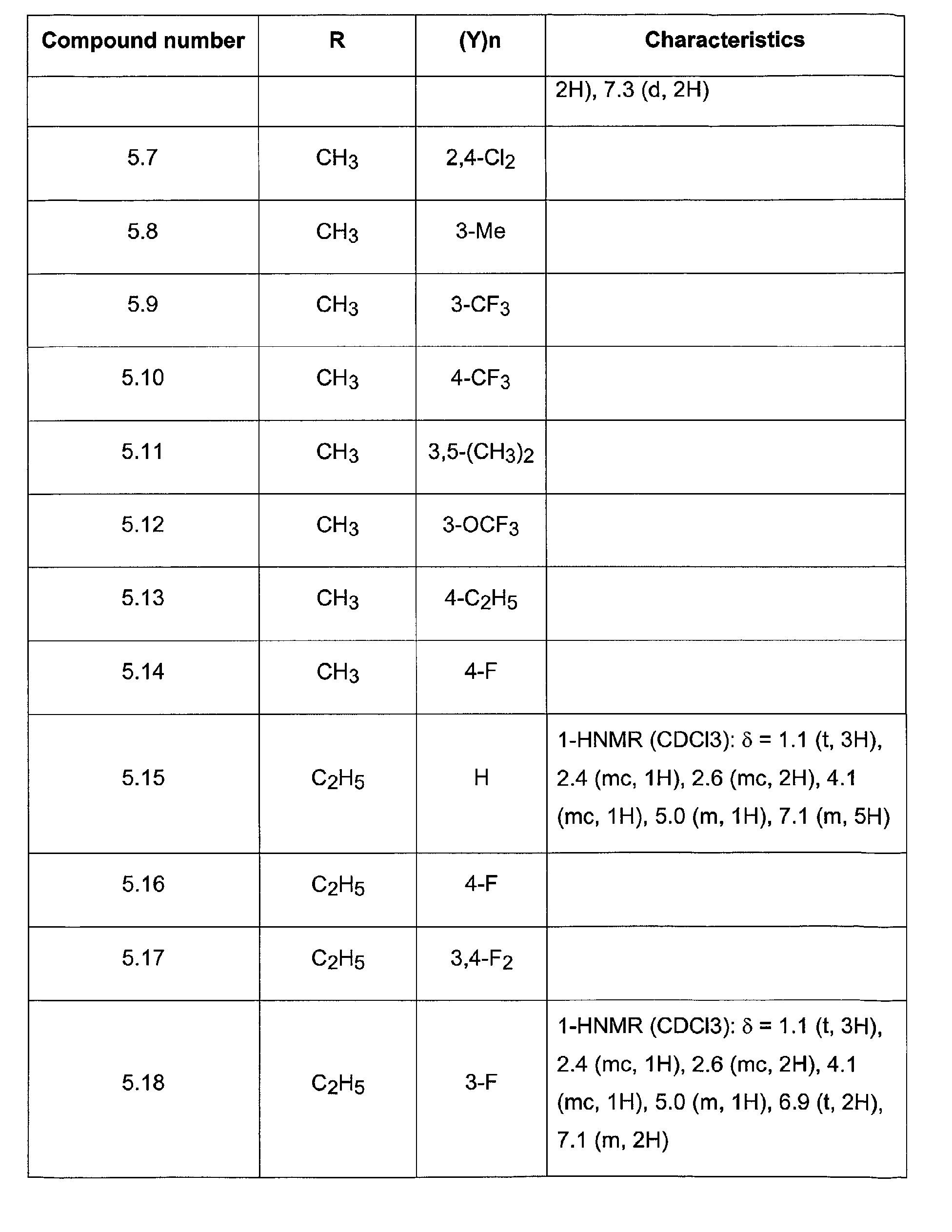

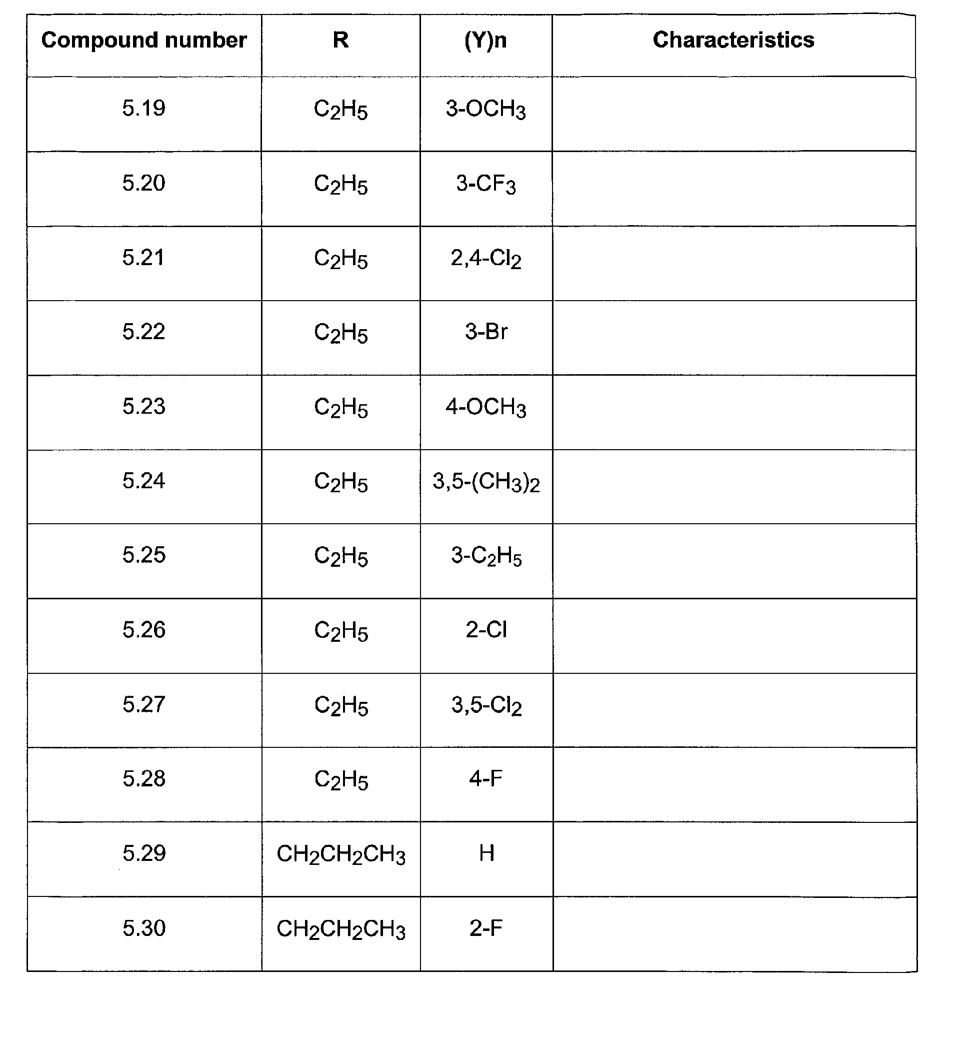

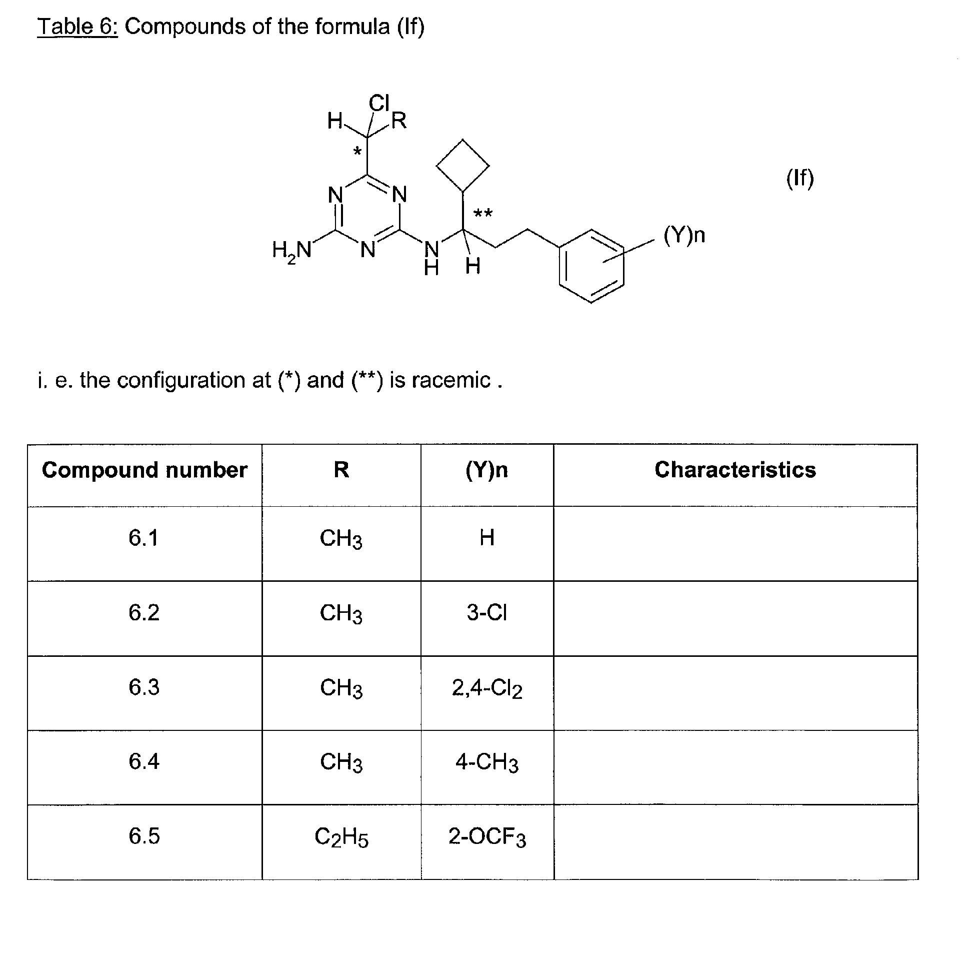

### B. Formulation examples

a) A dust is obtained by mixing 10 parts by weight of a compound of the formula (I) and 90 parts by weight of talc as inert substance and comminuting the mixture in a hammer mill.
b) A wettable powder which is readily dispersible in water is obtained by mixing 25 parts by weight of a compound of the formula (I), 64 parts by weight of kaolin-containing quartz as inert substance, 10 parts by weight of potassium lignosulfonate and 1 part by weight of sodium oleoylmethyltaurinate as wetter and dispersant and grinding the mixture in a pinned-disk mill.
c) A dispersion concentrate which is readily dispersible in water is obtained by mixing 20 parts by weight of a compound of the formula (I) with 6 parts by weight of alkylphenol polyglycol ether (@Triton X 207), 3 parts by weight of isotridecanol polyglycol ether (8 EO) and 71 parts by weight of paraffinic mineral oil (boiling range, for example, approx. 255 to above 277°C) and grinding the mixture in a ball mill to a fineness of below 5 microns.
d) An emulsifiable concentrate is obtained from 15 parts by weight of a compound of the formula (I), 75 parts by weight of cyclohexanone as solvent and 10 parts by weight of ethoxylated nonylphenol as emulsifier.
e) Water-dispersible granules are obtained by mixing
   75 parts by weight of a compound of the formula (I),
   10 " of calcium lignosulfonate,
   5 " of sodium lauryl sulphate,
   3 " of polyvinyl alcohol and
   7 " of kaolin,
   grinding the mixture in a pinned-disk mill and granulating the powder in a fluidised bed by spraying on water as granulation liquid.
f) Water-dispersible granules are also obtained by homogenising and precomminuting, on a colloid mill,
   25 parts by weight of a compound of the formula (I),
   5 " of sodium 2,2'-dinaphthylmethane-6,6'-disulfonate,
   2 " of sodium oleoylmethyltaurinate,
   1 part by weight of polyvinyl alcohol,
   17 parts by weight of calcium carbonate and
   50 " of water,
   subsequently grinding the mixture in a bead mill and atomising and drying the resulting suspension in a spray tower by means of a single-substance nozzle.

### C. Biological examples

### 1. Pre-emergence effect on weeds

Seeds or rhizome pieces of mono and dicotyledonous weed plants are placed in sandy loam soil in plastic pots and covered with soil. The compounds according to the invention which are formulated in the form of wettable powders or emulsion concentrates are then applied to the soil cover in the form of aqueous suspensions or emulsions at an application rate of 600 to 800 l of water/ha (converted), in various dosages.

After the treatment, the pots are placed in a greenhouse and kept under good growth conditions for the weeds. After the test plants had emerged, the damage to the plants or the negative effect on the emergence is visually scored after a test period of 3 to 4 weeks by comparison with untreated controls. As shown by the test results, the compounds according to the invention have good herbicidal pre-emergence activity against a broad spectrum of weed grasses and broad-leaved weeds.

For example, in the test, compound 1.1 and compound 2.1 showed very good herbicidal activity and, in addition, the racemic mixtures corresponding to compound 1.1 to 1.6, 1.15 to 1.19, 3.1, 2.1 to 2.6, 2.15 to 2.19 and 4.1 (i. e. racemic compound mixtures 5.1 to 5.6, 5.15 to 5.19, and 6.1) showed very good herbicidal activity pre-emergence against harmful plants such as Stellaria media, Lolium multiflorum, Amaranthus retroflexus, Sinapis alba, Avena sativa and Settaria viridis at an application rate of 1 kg or less of active substance per hectare.

When compared to one another with a view to herbicidal action, selectivity and activity spectrum, the R and S isomers in question show differences which can in each case be utilised in a targeted manner.

### 2. Post-emergence effect on weeds

Seeds or rhizome pieces of mono- and dicotyledonous weeds are placed in sandy loam soil in plastic pots, covered with soil and grown in a greenhouse under good growth conditions. Three weeks after sowing, the test plants are treated at the three-leaf stage. The compounds according to the invention, formulated as wettable powders or emulsion concentrates, are sprayed, at various dosages, onto the green parts of the plants at an application rate of 600 to 800 I of water/ha (converted). After the test plants had remained in the greenhouse for about 3 to 4 weeks under optimum growth conditions, the effect of the preparations is scored visually by comparison with untreated controls. The agents according to the invention also have good herbicidal activity post-emergence against a broad spectrum of economically important weed grasses and broad-leaved weeds.

For example, in the test, compound 1.1 and compound 2.1 showed very good herbicidal activity and, in addition, the racemic mixtures corresponding to compound 1.1 to 1.6, 1.15 to 1.19, 3.1, 2.1 to 2.6, 2.15 to 2.19 and 4.1 (i. e. racemic compound mixtures 5.1 to 5.6, 5.15 to 5.19, and 6.1) showed very good herbicidal activity post-emergence against harmful plants such as Sinapis alba, Echinochloa crus-galli, Lolium multiforum, Stellaria media, Cyperus iria, Amaranthus retroflexus, Settaria viridis and Avena sativa at an application rate of 1 kg or less of active substance per hectare.

When compared to one another with a view to herbicidal action, selectivity and activity spectrum, the R and S isomers in question show differences which can in each case be utilised in a targeted manner.

### 3. Action on harmful plants in rice

Transplanted and sown rice and also typical rice weeds (gramineous and broad-leaved) are cultivated in closed plastic pots in a greenhouse to the three-leaf stage (Echinochloa crus-galli 1.5-leaf) under paddy rice conditions (height of water: 2-3 cm). This is followed by treatment with the compounds according to the invention. For this purpose, the formulated active compounds are suspended, dissolved or emulsified in water and applied by pouring them into the dammed water around the test plants in different dosages.

After this treatment, the test plants are set up in the greenhouse under optimum growth conditions and are maintained in this way throughout the test period. About three weeks after application, evaluation is made by visual scoring of the damage to the plants by comparison with untreated controls. The compounds according to the invention show very good herbicidal activity against harmful plants.

For example, in the test, compound 1.1 and compound 2.1 showed very good herbicidal activity and, in addition, the racemic mixtures corresponding to compound 1.1 to 1.6, 1.15 to 1.19, 3.1, 2.1 to 2.6, 2.15 to 2.19 and 4.1 (i. e. racemic compound mixtures 5.1 to 5.6, 5.15 to 5.19, and 6.1) showed very good herbicidal activity against harmful plants which are typical for rice crops, for example Cyperus monti, Echinochloa crus-galli and Sagitaria pygmaea.

When compared to one another with a view to herbicidal action, selectivity and activity spectrum, the R and S isomers in question show differences which can in each case be utilised in a targeted manner.

### 4. Tolerance by crop plants

In further greenhouse experiments, seeds of a substantial number of crop plants and weeds are placed in sandy loam soil and covered with soil. Some of the pots are treated immediately as described under Section 1, and the remaining pots are placed in the greenhouse until the plants have developed two to three true leaves and then sprayed with various dosages of the substances of the formula (I) according to the invention, as described under Section 2. Visual scoring four to five weeks after the application and after the plants have been in the greenhouse reveals that the compounds according to the invention leave dicotyledonous crops such as soy, cotton, oilseed rape, sugar beet and potatoes unharmed even when high dosages of active ingredient were used by the pre- and post-emergence method. Moreover, some substances also spare Gramineae crops such as barley, wheat, rye, sorghum, corn or rice. Some of the compounds of the formula (I) have high selectivity, and they are therefore suitable for controlling undesirable vegetation in agricultural crops.

## Claims

1. A compound of the formula (I) or a salt thereof, 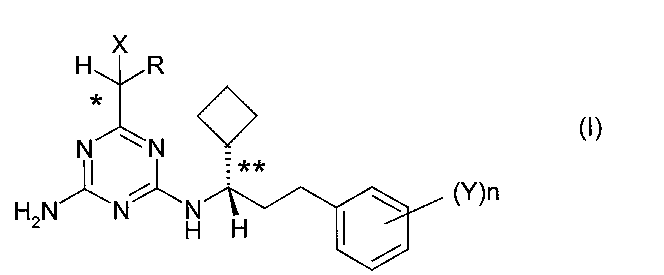 in which
the chiral carbon atom alpha to the amino group of the phenylalkylamino radical marked ** is in the S-configuration, as indicated in formula (I) whilst the chiral carbon atom to which the R and X radicals are bonded marked * can be in either the R or S configuration, and in which
R is (C₁-C₆)alkyl, (C₁-C₆)haloalkyl, [(C₁-C₆)alkoxy](C₁-C₆)alkyl, (C₂-C₆)alkenyl, (C₂-C₆)haloalkenyl, (C₁-C₆)alkoxy, (C₁-C₆)haloalkoxy or is (C₄-C₆)cycloalkenyl or is (C₃-C₆)cycloalkyl wherein the last two mentioned groups are unsubstituted or substituted by halogen or by one or two (C₁-C₄)alkyl groups,
X is halogen,
(Y)ₙ is n substituents Y, where Y in each case independently of one another is halogen, hydroxyl, amino, nitro, formyl, carboxyl, cyano, thiocyanato, aminocarbonyl or
(C₁-C₆)alkyl, (C₁-C₆)alkoxy, (C₁-C₆)alkylthio, mono(C₁-C₆)alkylamino, di(C₁-C₆)alkylamino, (C₂-C₆)alkenyl, (C₂-C₆)alkynyl, [(C₁-C₆)alkyl]carbonyl,
[( C₁-C₆)alkoxy]carbonyl, mono(C₁-C₆)alkylaminocarbonyl, di(C₁-C₆)alkylaminocarbonyl, N-(C₁-C₆)alkanoylamino or N-(C₁-C₆)alkanoyl-N-(C₁-C₆)alkylamino,
where each of the 13 last-mentioned radicals is unsubstituted or substituted,
or is (C₃-C₉)cycloalkyl, (C₃-C₉)cycloalkoxy, (C₃-C₉)cycloalkylamino, phenyl, phenoxy, phenylthio, phenylcarbonyl, heterocyclyl, heterocyclyloxy, heterocyclylthio or heterocyclylamino,
where each of the 11 last-mentioned radicals is unsubstituted or substituted,
or two adjacent radicals Y together are a fused-on ring having 4 to 6 ring atoms, the ring being carbocyclic or additionally containing hetero ring atoms from the group consisting of O, S and N and being unsubstituted or substituted by one or more radicals from the group consisting of halogen, (C₁-C₆)alkyl and oxo, and
n is 0, 1, 2, 3, 4 or 5.

2. A compound according to claim 1 **characterised in that**
R is (C₁-C₆)alkyl, (C₁-C₆)haloalkyl, [(C₁-C₆)alkoxy](C₁-C₆)alkyl, (C₂-C₆)alkenyl, (C₂-C₆)haloalkenyl, (C₁-C₆)alkoxy, (C₁-C₆)haloalkoxy or is (C₄-C₆)cycloalkenyl or is (C₃-C₆)cycloalkyl wherein the last two mentioned groups are unsubstituted or substituted by halogen or by one or two (C₁-C₄)alkyl groups,
X is halogen,
(Y)ₙ is n substituents Y, where Y in each case independently of one another is halogen, hydroxyl, amino, nitro, formyl, carboxyl, cyano, thiocyanato, aminocarbonyl or
(C₁-C₆)alkyl, (C₁-C₆)alkoxy, (C₁-C₆)alkylthio, mono(C₁-C₆)alkylamino, di(C₁-C₆)alkylamino, (C₂-C₆)alkenyl, (C₂-C₆)alkynyl, [(C₁-C₆)alkyl]carbonyl, [(C₁-C₆)alkoxy]carbonyl, mono(C₁-C₆)alkylaminocarbonyl, di(C₁-C₆)alkylaminocarbonyl, N-(C₁-C₆)alkanoylamino or N-(C₁-C₆)alkanoyl-N-(C₁-C₆)alkylamino,
where each of the 13 last-mentioned radicals is unsubstituted or substituted by one or more radicals selected from the group consisting of halogen, hydroxyl, amino, nitro, formyl, carboxyl, cyano, thiocyanato, (C₁-C₆)alkoxy, (C₁-C₆)haloalkoxy, (C₁-C₆)alkylthio, (C₁-C₆)halo- alkylthio, mono(C₁-C₆)alkylamino, di(C₁-C₆)alkylamino, (C₃-C₉)cycloalkyl, (C₃-C₉)cycloalkylamino, [(C₁-C₆)alkyl]carbonyl, [(C₁-C₆)alkoxy]carbonyl, aminocarbonyl, mono(C₁-C₆)alkylamino carbonyl, di(C₁-C₆)alkylaminocarbonyl, phenyl, phenoxy, phenylthio, phenylcarbonyl, heterocyclyl, heterocyclyloxy, heterocyclylthio and heterocyclylamino,
where each of the 8 last-mentioned radicals is unsubstituted or substituted by one or more substituents selected from the group consisting of halogen, nitro, cyano, (C₁-C₆)alkyl, (C₁-C₆)alkoxy, (C₁-C₆)alkylthio, (C₁-C₆)haloalkyl, C₁-C₆)haloalkoxy, formyl, (C₁-C₆)alkyl-carbonyl and (C₁-C₆)alkoxy-carbonyl,
or is (C₃-C₉)cycloalkyl, (C₃-C₉)cycloalkoxy, (C₃-C₉)cycloalkylamino, phenyl, phenoxy, phenylthio, phenylcarbonyl, heterocyclyl, heterocyclyloxy, heterocyclylthio or heterocyclylamino,
where each of the 11 last-mentioned radicals is unsubstituted or substituted by one or more radicals selected from the group consisting of halogen, hydroxyl, amino, nitro, formyl, carboxyl, cyano, thiocyanato, (C₁-C₆)alkyl, (C₁-C₆)haloalkyl, (C₁-C₆)alkoxy, (C₁-C₆)haloalkoxy, (C₁-C₆)alkylthio, (C₁-C₆)haloalkylthio, mono(C₁-C₆)alkylamino, di(C₁-C₆)alkylamino, (C₃-C₉)cycloalkyl, [(C₁-C₆)alkyl]carbonyl, [(C₁-C₆)alkoxy]carbonyl, aminocarbonyl, mono(C₁-C₆)alkylaminocarbonyl and di(C₁-C₆)alkylaminocarbonyl,
or two adjacent radicals Y together are a fused-on ring having 4 to 6 ring atoms, the ring being carbocyclic or containing hetero ring atoms from the group consisting of O, S and N and being unsubstituted or substituted by one or more radicals from the group consisting of halogen, (C₁-C₆)alkyl and oxo, and
n is 0, 1, 2, 3, 4 or 5.

3. A compound according to claim 1 **characterised in that**
R is (C₁-C₆)alkyl, (C₁-C₆)haloalkyl, [(C₁-C₆)alkoxy](C₁-C₆)alkyl, (C₂-C₆)alkenyl, (C₂-C₆)haloalkenyl, (C₁-C₆)alkoxy, (C₁-C₆)haloalkoxy or is (C₄-C₆)cycloalkenyl or is (C₃-C₆)cycloalkyl wherein the last two mentioned groups are unsubstituted or substituted by halogen or by one or two (C₁-C₄)alkyl groups
X is halogen, and
(Y)ₙ is n substituents Y, where Y in each case independently of one another is preferably halogen, hydroxyl, amino, nitro, formyl, carboxyl, cyano, thiocyanato, aminocarbonyl or
(C₁-C₆)alkyl, (C₁-C₆)alkoxy, (C₁-C₆)alkylthio, mono(C₁-C₆)alkylamino, di(C₁-C₆)alkylamino, (C₂-C₆)alkenyl, (C₂-C₆)alkynyl, [(C₁-C₆)alkyl]carbonyl, [(C₁-C₆)alkoxy]carbonyl, mono(C₁-C₆)alkyl-aminocarbonyl, di(C₁-C₄)alkylaminocarbonyl, N-(C₁-C₆)alkanoylamino or N-(C₁-C6)alkanoyl-N-(C₁-C₆)alkylamino,
where each of the 13 last-mentioned radicals is unsubstituted or substituted by one or more radicals selected from the group consisting of halogen, hydroxyl, amino, nitro, formyl, carboxyl, cyano, thiocyanato, (C₁-C₆)alkoxy, (C₁-C₆)haloalkoxy and (C₁-C₆)alkylthio,
or is (C₃-C₉)cycloalkyl, (C₃-C₉)cycloalkoxy, (C₃-C₉)cycloalkylamino, phenyl, phenoxy, phenylthio, phenylcarbonyl, heterocyclyl, heterocyclyloxy, heterocyclylthio or heterocyclylamino,
where each of the 11 last-mentioned radicals is unsubstituted or substituted by one or more radicals selected from the group consisting of halogen, hydroxyl, amino, nitro, formyl, carboxyl, cyano, thiocyanato, (C₁-C₆)alkyl, (C₁-C₆)haloalkyl, (C₁-C₆)alkoxy, (C₁-C₆)haloalkoxy and (C₁-C₆)alkylthio,
or two adjacent Y radicals together are a fused-on ring having 4 to 6 ring atoms, the ring being carbocyclic or containing hetero ring atoms from the group consisting of O, S and N and being unsubstituted or substituted by one or more radicals from the group consisting of halogen, (C₁-C₄)alkyl and oxo, and
n is 0, 1, 2, 3, 4 or 5.

4. A compound according to claim 1 **characterised in that**
R is (C₁-C₆)alkyl, (C₁-C₆)haloalkyl, (C₂-C₆)alkenyl or
(C₂-C₆)haloalkenyl and,
X is fluorine or chlorine or bromine, and
(Y)ₙ is n substituents Y, where Y in each case independently of one another is preferably halogen, hydroxyl, amino, nitro, formyl, carboxyl, cyano, thiocyanato, aminocarbonyl or
(C₁-C₆)alkyl, (C₁-C₆)alkoxy, (C₁-C₆)alkylthio, mono(C₁-C₆)alkylamino, di(C₁-C₆)alkylamino, (C₂-C₆)alkenyl, (C₂-C₆)alkynyl, [(C₁-C₆)alkyl]carbonyl, [(C₁-C₆)alkoxy]carbonyl, mono(C₁-C₆)alkyl-aminocarbonyl, di(C₁-C₆)alkylaminocarbonyl, N-(C₁-C₆)alkanoylamino or N-(C₁-C₆)alkanoyl-N-(C₁-C₆)alkylamino,
where each of the 13 last-mentioned radicals is unsubstituted or substituted by one or more radicals selected from the group consisting of halogen, hydroxyl, amino, nitro, formyl, carboxyl, cyano, thiocyanato, (C₁-C₆)alkoxy, (C₁-C₆)haloalkoxy and (C₁-C₆)alkylthio
or is (C₃-C₉)cycloalkyl, and
n is 0, 1, 2, 3, 4 or 5.

5. A compound according to claim 1 **characterised in that**
R is (C₁-C₆)alkyl, (C₁-C₆)haloalkyl, (C₂-C₆)alkenyl or
(C₂-C₆)haloalkenyl, and
X is fluorine or chlorine, and
(Y)ₙ is n substituents Y, where Y in each case independently of one another is preferably halogen, hydroxyl, amino, nitro, formyl, carboxyl, cyano, thiocyanato, aminocarbonyl or
(C₁-C₆)alkyl, (C₁-C₆)alkoxy, (C₁-C₆)alkylthio, mono(C₁-C₆)alkylamino, di(C₁-C₆)alkylamino, (C₂-C₆)alkenyl, (C₂-C₆)alkynyl, [(C₁-C₆)alkyl]carbonyl, [(C₁-C₆)alkoxylcarbonyl, mono(C₁-C₆)alkyl-aminocarbonyl, di(C₁-C₆)alkylaminocarbonyl, N-(C₁-C₆)alkanoylamino or N-(C₁-C₆)alkanoyl-N-(C₁-C₆)alkylamino,
where each of the 13 last mentioned radicals is unsubstituted or substituted by halogen,
or is (C₃-C₉)cycloalkyl, and
n is 0, 1, 2, 3, 4 or 5.

6. A compound of the formula (I) according to claim 1 **characterised in that**
R is (C₁-C₆)alkyl, and
X is fluorine or chlorine, and
(Y)ₙ is n substituents Y, where Y in each case independently of one another is preferably halogen, hydroxyl, nitro, cyano, (C₁-C₆)alkyl, (C₁-C₆)alkoxy or (C₁-C₆)alkylthio,
where each of the 3 last mentioned radicals is unsubstituted or substituted by halogen
or is (C₃-C₉)cycloalkyl, and
n is 0,1 ,2,or 3.

7. A process for the preparation of a compound of formula (I), or a salt thereof, according to any one of claims 1 to 6 which comprises:
a) reacting an optically active compound of the formula (II), 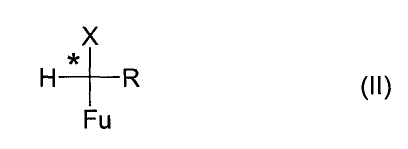 in which Fu is a functional group selected from the group consisting of carboxylic esters, carboxylic orthoesters, carbonyl chlorides, carboxamides, carboxylic anhydrides and trichloromethyl,
with a compound of the formula (III) or an acid addition salt thereof 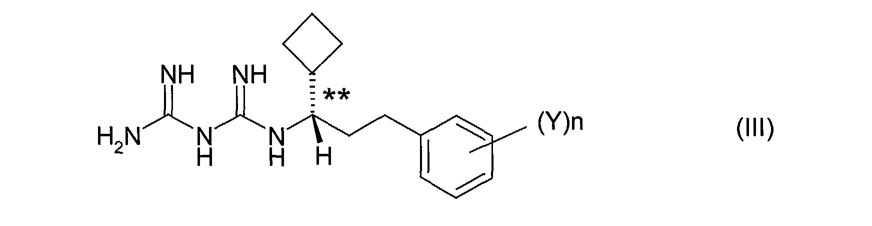 or
b) reacting a compound of the formula (IV), 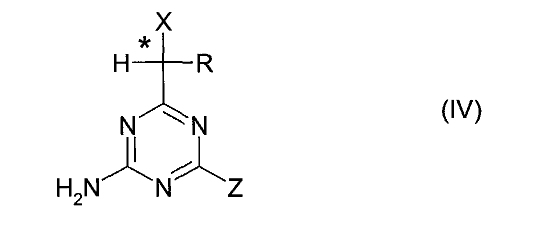 in which Z is an exchangeable radical or a leaving group,
with an amine of the formula (V) or an acid addition salt thereof, 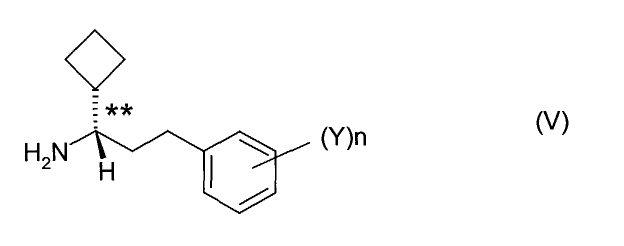 where in the formulae (II), (III), (IV) and (V) of the process variants a) and b) the radicals R, X and Y, the integer n and the configuration of the carbon atoms marked in the formulae with (*) or (**), are as defined in formula (I), or
c) reacting compounds of the formula (II) with compounds of formula (III), or compounds of the formula (IV) with compounds of the formula (V), wherein either or both of the reactants is used in the form of a mixture of enantiomers with respect to the carbon atoms (*) or (**) that is different from that required in the final compound of the formula (I), and then separating the resulting mixture of products to yield a compound of the formula (I) in which the chiral carbon atoms (*) and/or (**) are in the desired form.

8. A herbicidal or plant growth regulating composition **characterised in that** it comprises one or more compounds of the formula (I) or salts thereof as defined any one of claims 1 to 6 and formulation auxiliaries which are customary in crop protection.

9. A method of controlling harmful plants or regulating the growth of plants **characterised in that** it comprises applying an effective amount of one or more compounds of the formula (I) or salts thereof as defined in any one of claims 1 to 6 to the plants to plant seeds or to the area under cultivation.

10. The use of compounds of the formula (I) or salts thereof as defined in any one of claims 1 to 6 as herbicides or plant growth regulators.

11. The use according to claim 10 **characterised in that** the compound of the formula (I) or the salt thereof is used for controlling harmful plants or for regulating the growth of plants in crops of useful or ornamental plants.

12. The use according to claim 11 **characterised in that** the crop plants are transgenic crop plants.
